(19)

Europäisches
Patentamt
European
Patent Office
Office européen
des brevets

(11) **EP 2 737 049 B1**

(12) **EUROPEAN PATENT SPECIFICATION**

(45) Date of publication and mention
of the grant of the patent:
**19.12.2018 Bulletin 2018/51**

(51) Int Cl.:
***C12N 1/12*** *(2006.01)*       ***C12P 7/64*** *(2006.01)*
***C12M 1/00*** *(2006.01)*

(21) Application number: **12740727.8**

(22) Date of filing: **18.07.2012**

(86) International application number:
**PCT/NL2012/000054**

(87) International publication number:
**WO 2013/015680 (31.01.2013 Gazette 2013/05)**

(54) **METHODS FOR OBTAINING ALGAL BIOMASS**

VERFAHREN ZUR GEWINNUNG VON ALGENBIOMASSE

PROCÉDÉS PERMETTANT D'OBTENIR UNE BIOMASSE ALGALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.07.2011 NL 2007181**

(43) Date of publication of application:
**04.06.2014 Bulletin 2014/23**

(73) Proprietors:
• **Unilever N.V.**
**3013 AL Rotterdam (NL)**
Designated Contracting States:
**HR IS NO PT RS TR**
• **Unilever BCS Europe B.V.**
**3013 AL Rotterdam (NL)**
Designated Contracting States:
**AL AT BE BG CH CZ DE DK EE ES FI FR GR HU
IT LI LT LU LV MC MK NL PL RO SE SI SK SM**
• **Unilever PLC**
**London EC4Y 0DY (GB)**
Designated Contracting States:
**MT**
• **Unilever BCS Limited**
**London, EC4Y 0DY (GB)**
Designated Contracting States:
**CY GB IE**

(72) Inventors:
• **SEVERINO DO ROSÁRIO DE QUINTANILHA DOS
SANTOS,**
**NL-8934 CJ Leeuwarden (NL)**
• **LAMERS, Packo Petrus**
**NL-8934 CJ Leeuwarden (NL)**

• **JANSSEN, Marcel**
**NL-8934 CJ Leeuwarden (NL)**
• **WIJFFELS, René Hubertus**
**NL-8934 CJ Leeuwarden (NL)**

(74) Representative: **EP&C**
**P.O. Box 3241**
**2280 GE Rijswijk (NL)**

(56) References cited:
• **LEVINE ROBERT B ET AL: "Neochloris
oleoabundans grown on anaerobically digested
dairy manure for concomitant nutrient removal
and biodiesel feedstock production", BIOMASS
AND BIOENERGY, vol. 35, no. 1, January 2011
(2011-01), pages 40-49, XP002670149, ISSN:
0961-9534**
• **GARDNER ROBERT ET AL: "Medium pH and
nitrate concentration effects on accumulation of
triacylglycerol in two members of the
chlorophyta", JOURNAL OF APPLIED
PHYCOLOGY, vol. 23, no. 6, 3 December 2010
(2010-12-03), pages 1005-1016, XP002670150,
ISSN: 0921-8971**
• **JAMES B. GUCKERT ET AL: "TRIGLYCERIDE
ACCUMULATION AND FATTY ACID PROFILE
CHANGES IN CHLORELLA (CHLOROPHYTA)
DURING HIGH pH-INDUCED CELL CYCLE
INHIBITION1", JOURNAL OF PHYCOLOGY, vol.
26, no. 1, 1 March 1990 (1990-03-01), pages 72-79,
XP55039043, ISSN: 0022-3646, DOI:
10.1111/j.0022-3646.1990.00072.x**

**(Cont. next page)**

- SIAUT MAGALI ET AL: "Oil accumulation in the model green alga Chlamydomonas reinhardtii: characterization, variability between common laboratory strains and relationship with starch reserves", BMC BIOTECHNOLOGY, BIOMED CENTRAL LTD. LONDON, GB, vol. 11, no. 1, 21 January 2011 (2011-01-21), page 7, XP021087838, ISSN: 1472-6750, DOI: 10.1186/1472-6750-11-7
- SANTOS A M ET AL: "Growth of oil accumulating microalga Neochloris oleoabundans under alkaline-saline conditions", BIORESOURCE TECHNOLOGY, vol. 104, January 2012 (2012-01), pages 593-599, XP002670151, DOI: DOI:10.1016/J.BIORTECH.2011.10.084
- Joel C. Goldman ET AL: "The effect of pH in intensive microalgal cultures. I. Biomass regulation", JOURNAL OF EXPERIMENTAL MARINE BIOLOGY AND ECOLOGY, vol. 57, no. 1, 1 January 1982 (1982-01-01), pages 1-13, XP055328803, NL ISSN: 0022-0981, DOI: 10.1016/0022-0981(82)90140-X
- Yuan-Kun Lee ET AL: "CO2 Absorption Rate in an Algal Culture: Effect of pH", J Chem Tech Biotechnol, vol. 34B, 1 January 1984 (1984-01-01), pages 28-32, XP055328733,
- Tania Mazzuca Sobczuk ET AL: "Potential fuel oils from the microalga Choricystis minor", Journal of Chemical Technology and Biotechnology, vol. 85, no. 1, 7 October 2009 (2009-10-07), pages 100-108, XP055416848, ISSN: 0268-2575, DOI: 10.1002/jctb.2272
- R Vazquez-Duhalt ET AL: "Oil production from microalgae under saline stress" In: "Biomass for Energy and Industry, 5th E.C. Conference, Volume 1 Policy, Environment, Production and Harvesting", 1 January 1990 (1990-01-01), Elsevier, England, XP055417020, ISBN: 978-1-85166-492-4 pages 1547-1552,

**Description**

The present invention relates to a method for obtaining algal biomass. In particular, the invention relates to obtaining algal biomass, e.g. lipids, such as fatty acids comprised by algal cells, by cultivating photoautotrophic freshwater micro-algae. For example, biomass from (micro-)algae is obtained for use as biofuels, fertilizer or nutrition.

It is known in the art to cultivate (micro-)algae for obtaining algal biomass for instance for use as biofuels, fertilizer, feedstock or nutrition. For example, (micro-) algae are cultivated to obtain lipids, proteins, polysaccharides and certain vitamins. The yield of such substances is limited in conventional methods. Specifically, lipid-rich microalgae are one of the most promising organisms for a sustainable production of biofuels. Yet, there are still some challenges with respect to process optimization. One of those challenges is to improve the lipid yields on light by maximization of biomass and lipid productivities. Maximal productivity of photoautotrophic microalgae is attainable when light is the only limitation for biomass growth, which requires that all necessary nutrients are present and that temperature, salinity and pH are optimal. Under such optimal conditions, the efficiency with which light energy is used for microalgae growth depends largely on the incident light intensity, the optical path length and the biomass density. Microalgal biofuels production may require natural sunlight and photobioreactors with a non-adjustable optical path length. Hence, the most suitable parameter that can be controlled to achieve an optimal photosynthetic efficiency throughout the entire production period is the biomass density. Such control is readily attainable by adjusting the dilution rate of the system. Implementing such a control strategy requires detailed knowledge on the relations between incident light intensity, biomass density and photosynthetic efficiency. Another matter concerning the existing microalgal biofuels production technologies is the high energy input for the transfer of carbon dioxide ($CO_2$) from gas to liquid. A promising option for reducing this energy cost in photobioreactors is to optimize the CO2 transfer in a separate gas transfer unit at elevated pH. In this way, the growth medium could be saturated with dissolved bicarbonate ($HCO_3^-$) before entering the cultivation system. Besides for a 2-stage process, working at high pH is also advantageous for traditional single-stage microalgae cultivation processes. $CO_2$ dissolution can be enhanced at high pH by direct reaction of $CO_2$ with hydroxide (OH-) in the liquid boundary layer surrounding the gas bubbles.
This results in a fast formation of $HCO_3^-$ which serves as an extra carbon carrier over the boundary layer. Bicarbonate would then serve as the major source of inorganic carbon and less energy would need to be invested in CO2 gas-liquid transfer inside the photobioreactor.
Mazzuca Sobczuk et al. (Journal of Chemical Technology and Biotechnology, 85(1):100-108, October 2009) discloses the application of stress factors of nitrogen depletion, elevated pH and/or high salinity during the culture of the freshwater microalga Choricystis minor. Vazquez-Duhalt R. et al. (Oil production from microalgae under saline stress, In: G. Grassi, G. Gosse, G. dos Santos: "Biomass for Energy and Industry, 5th E.C. Conference, Volume 1, Policy, Environment, Production and Harvesting", 1990, pages 1547-1552) discloses the cultivation of micro-algae under saline stress at pH 7.5 and 0.25-0.75 M NaCl.

The goals of the present invention, amongst other goals, are to overcome or reduce the above mentioned disadvantages and to provide an improved method for obtaining algal biomass by cultivating algae.

This objective is achieved with a method for obtaining algal biomass with increased lipid accumulation, comprising the step of culturing photoautotrophic freshwater micro-algae in a culturing medium having a pH above 9, wherein lipid accumulation is increased compared to cultivation of such algal biomass at a pH below 8, wherein the medium is a saline medium, wherein the cultivating medium comprising NaCl in a concentration of at least 200 mM, wherein the algae are cultivated under conditions of nutrient limitation, wherein the nutrient limitation conditions comprise nitrogen limitation.

Surprisingly, cultivating photoautotrophic freshwater micro-algae in a culturing medium having a pH above 9, and wherein the medium is a saline medium, wherein the cultivating medium comprising NaCl in a concentration of at least 200 mM, wherein the algae are cultivated under conditions of nutrient limitation, wherein the nutrient limitation conditions comprise nitrogen limitation alters the composition of the algal biomass. The lipid content of the algae increases. In other words the ratio of lipids in the algal biomass increases. Furthermore, the rate of this increase is relatively high. In other words, the ratio of accumulated lipids in the algal biomass is increased, and this higher ratio is achieved in a relative short period of time.

For example, the algae are cultivated in a medium with a substantially constant pH level above 9. Alternatively, the algae are grown in a first step at conventional pH levels until a constant growth rate has been established. In a next step the pH of the medium is elevated to achieve lipid accumulation.

A further advantage is that by cultivating algae at a pH higher than 9, the $CO_2$ transfer to the medium is improved. When cultivating algae according to conventional methods, substantial energy is required for the supply of carbon dioxide ($CO_2$) to large-scale cultivation systems by means of gas transfer, which is a big restriction for the potential uses of these systems. During (micro-) algal cultivation, $CO_2$ is usually introduced to the system by $CO_2$ enrichment of the inlet air flow, but it is also envisioned to use $CO_2$-rich gasses as for example flue gas. However, a considerable amount of $CO_2$ which fails to be transferred into the liquid phase will still be wasted to the effluent gas.

By using a medium with a pH above 9, the $CO_2$ uptake of a medium is increased.

In a fluid with dissolved $CO_2$, an equilibrium exists between dissolved bicarbonate ($HCO_3^-$) and $CO_2$ and, in the case of a high pH, also dissolved carbonate ($CO_3^{2-}$). When (micro-)algae take up $CO_2$ from the fluid, $CO_2$ is replenished by dissociation of bicarbonate in $OH^-$ and $CO_2$. This increases the pH of the fluid. If no $CO_2$ is added to compensate for the carbon dioxide uptake by algae, the pH will continue to increase, even above 11 and finally only carbonate will remain in the fluid.

Working at conventional pH levels (< 8), the supplied carbon dioxide gas has to react with water ($H_2O$) to form $H_2CO_3$, which subsequently (partially) dissociates to $H^+$ and $HCO^{3-}$. This is a relatively slow chemical reaction.

In the method according to the invention, due to the increased pH level, the $CO_2$ can react directly with the $OH^-$ to form $HCO^{3-}$.

This reaction is faster than the above described reaction involving dissociation of $H_2CO_3^-$. When using a pH above 9 according to the method of the invention, diffusion of $HCO^{3-}$ at the fluid/gas boundary layer of the gas bubbles contributes to the transfer of the substances. In contrast, in conventional methods with a lower pH, only $CO_2$ diffuses at the boundary layer.

Due to the relative fast transfer of $CO_2$ to the medium in the method according to the invention, the $CO_2$ gas can be supplied to the medium at a lower rate. This significantly reduces energy consumption.

Furthermore, the cultivation medium can comprise a relatively large concentration of inorganic carbon as bicarbonate ($HCO^{3-}$), which provides a $CO_2$ source. Therefore, less $CO_2$ has to supplied to the medium by an external source, such as a gas supply.

For example, in the method according to the invention, bicarbonate is added to the medium prior to supplying the medium to a cultivation system. The energy necessary for $CO_2$ transfer from the gas to the liquid phase could thus be reduced by optimizing $CO_2$ transfer in a separate gas transfer unit at elevated pH (>9.0).

According to the invention, cultivation can be batchwise or continuous.

For example, lipids comprise fatty acids.

In a preferred embodiment according to the invention, the pH of the medium is in the range of 9-11 and most preferably 10-11. In the context of the present invention, the pH values and ranges given are to be understood as an indication of the activity of the (solvated) hydrogen ion. The abbreviation p[H], which measures the hydrogen ion concentration is called pH. Solutions with a pH less than 7 are said to be acidic and solutions with a pH greater than 7 are basic or alkaline. The pH scale is traceable to a set of standard solutions whose pH is established by international agreement. Primary pH standard values are determined using a concentration cell with transference, by measuring the potential difference between a hydrogen electrode and a standard electrode such as the silver chloride electrode. Measurement of pH for aqueous solutions can be done with a glass electrode and a pH meter, or using indicators. In the method of the present invention, the pH is above 9, advantageously above 9.5. In the context of the present invention, the pH is below 12, advantageously below 11.5, more advantageously below 11. The pH can have any values between 9 and 11.

Experiments conducted by the inventors show good results using in particular a pH of about 10. The term "about" is to be understood as within the experimental measurement error margin. Accordingly, all the values and ranges in the context of the present invention may vary around one value or range within a few tenth of pH unit, depending on the precision of the measuring instrument.

In a preferred embodiment the algal biomass comprises algal cells, preferably lipids comprised by the algal cells.

For example, lipids are obtained for use as biofuels or feedstock. Surprisingly, in particular lipid accumulation is increased in algal biomass when cultivating algae in a medium having a pH above 9, equal to, or above 9.5, equal to, or above 10,and wherein the medium is a saline medium, wherein the cultivating medium comprising NaCl in a concentration of at least 200 mM, wherein the algae are cultivated under conditions of nutrient limitation, wherein the nutrient limitation conditions comprise nitrogen limitation. The pH can have any values chosen from the group 9, 9.5, 10, 10.5 and 11. The final concentration of lipids is higher than in conventional methods and this level is achieved quicker.

According to a further preferred embodiment, the algae are lipid-rich algae. Certain types of algae are known for their intrinsic high level of lipids. By cultivating these algae using the method of the invention, the lipid content of the algae can be increased even further.

In the method according to the invention, the algae are photoautotrophic freshwater micro-algae.

Preferably, the photoautotrophic freshwater micro-algae are lipid-rich micro-algae, when the method is aimed at harvesting lipids from algae.

In fact, due to their high photosynthetic efficiency, high biomass productivity and fast growth rates in comparison to higher plants, lipid-rich photoautotrophic freshwater micro-algae can be considered as potentially one of the most efficient biological oil producers.

In a preferred embodiment, the algae comprise algae of the species *Neochloris oleoabundans*.

*Neochloris oleoabundans* is capable of producing oil bodies composed of triacylglycerides (TAG) at a level of up to 80% of its total lipid composition. Most of those lipids are composed of saturated fatty acids, perfectly suitable for the production of biofuels.

Surprisingly, algae of the *Neochloris oleoabundans* species are able to accumulate lipids under high pH (> 9) conditions. Furthermore, the lipid content of the algae increases as compared to cultivation in a lower pH medium.

In the method according to the invention, the medium is a saline medium. By "saline medium" is to be understood saline aqueous medium, wherein the cultivating medium comprises NaCl in a concentration of at least 200 mM.

Good results have been obtained by the method of the present invention when the salinity is in the range 0.7 to 7%, preferably 1% to 6%, more preferably in the range 2 to 5%, most preferably in the range 3% to 5%. By the term "salinity" is to be understood the weight% of salt relative to the total weight of aqueous medium.

Surprisingly, the growth rate of algae under high pH conditions turns out to be higher when using a saline aqueous medium than using, for example, a freshwater based medium.

In particular, an alkaline (pH > 9) and saline aqueous medium is used to grow algae of the species *Neochloris oleoabundans.*

Preferably, the medium comprises seawater and/or artificial seawater.

This is in particular an advantage when cultivating these algae in a location where freshwater is scarce. Using the method of the invention seawater can be used instead of freshwater.

In a preferred embodiment of the method according to the invention, the cultivating medium comprises alkali metal chlorine salts being NaCl in a concentration of at least 200, 270 or 400 mM.

Freshwater can be defined as water with less than 500 parts per million of dissolved salts.

Preferably, the sum of the concentrations of Na, $CO_3^{2-}$ and $HCO_3^{3-}$ is about 350-500 mM, more preferably 420 mM.

In a preferred embodiment, the pH of the culturing medium is regulated by equilibrium of carbon dioxide, bicarbonate and carbonate.

By culturing the algae in a medium comprising bicarbonate ($HCO_3^-$) and/or carbonate ($CO_3^{2-}$), the alkalinity of the medium can be regulated by the distribution of the bicarbonate and carbonate species ($HCO_3$ and $CO_3^{2-}$).

Bicarbonate and carbonate salts determine the buffering capacity of the medium. By providing them at high enough levels to obtain this buffer function, the necessary $CO_2$ for algal growth is provided, while preventing pH variations. Preferably, $CO_2$ is supplied to compensate for $CO_2$ uptake.

In a further embodiment the bicarbonate and carbonate ions are present in an amount sufficient to buffer the medium at a pH of at least 9, 9.5, 10, 10.5 or 11.

For example, bicarbonate and carbonate is provided by adding $NaHCO_3$ and $Na_2CO_3$. For example bicarbonate and carbonate are each present in a concentration of at least 10, 30, 50, 60, 75, 90, 100, 150, 210, 240 or at least 270 mM. Advantageously, bicarbonate and carbonate anions are each present at a concentration in the range 10 to 150 mM, more advantageously, 50 to 100 mM. More advantageously, the concentration of bicarbonate or carbonate is 75mM.

Preferably the medium is saturated with the bicarbonate and/or carbonate.

The $CO_2$ transfer can take place prior to supplying it to a system for growing algae, or during the growth stage. In traditional systems where $CO_2$ is supplied in the actual growth stage, the efficiency of $CO_2$ transfer can be increased by the method according to the invention.

In a preferred embodiment, the carbon dioxide is supplied in a gas which comprises 0.01%-20% (v/v) carbon dioxide, preferably 0.038-20% (v/v), more preferably 0.038-4% (v/v).

For example, air is supplied which has a carbon dioxide content of about 0.038 % (v/v). In another example, a gas comprising 0.01-4 % carbon dioxide is supplied. In another example, a flue gas comprising 10-20 % (v/v) carbon dioxide is supplied.

By recirculating the gas, the carbon dioxide content may decrease with respect to the initial carbon dioxide content.

In the method according to the invention, the algae are cultivated under conditions of nutrient limitation, wherein the nutrient limitation conditions comprise nitrogen limitation.

For example, the method comprises providing a nitrogen-poor or nitrogen-free medium after a constant growth rate has been established.

Nitrogen limitation can be defined as the condition wherein the amount of nitrogen is a growth limiting factor. Nitrogen starvation can be defined as the condition wherein the amount of nitrogen is the dominant growth limiting factor.

In particular, the method according to the invention comprises the combination of cultivating algae of the *Neochloris oleoabundans* species in a cultivating medium having a pH above 9 and applying a nutrient limiting condition, preferably nitrogen limitation.

Experiments of the inventors show that at high pH and under nitrogen limiting conditions the most abundant fatty acids in *Neochloris oleoabundans* are C16:0, C18:1 and C18:2, which together account for more than 90% of the total fatty acid content. These fatty acids form a suitable feedstock for the production of biofuels, coatings and surface active agents. The fraction of these fatty acids that is present in triglycerides can also serve as a replacement of the traditional crop oil that is used in food industry.

Preferably the algae are of the species *Neochloris sp., Neochloris oleoabundans, Ettlia sp.* or *Ettlia texensis.*

Alternatively, the algae are of the species *Scenedesmus obliquus, Chlorella vulgaris, Chlorella zofingiensis, Dunaliella tertiolecta, Dunaliella salina, Tetraselmis suecica, Parietochloris incise, Nannochloris sp., Nannochloropsis sp., Nannochloropsis oculata, Nannochloropsis salina, Isochrysis galbana, Porphyridium cruentum, Phaeodactylum tricornutum, Chaeotoceros muelleri, Chaeotoceros calcitrans, Chaeotoceros gracilis* or *Monodus subterraneus.*

In a preferred embodiment of the method according to the invention, the cultivating medium comprises ions of a soluble salt, the concentration being such that no significant precipitation of the ions at the given pH level occurs, i.e. the saturation point is not reached.

Algal growth depends on an adequate supply of nutrients. These nutrients are usually highly soluble salts which dissociate in ions and are either present in high concentrations, as in the case of magnesium ($Mg^{2+}$) and calcium ($Ca^{2+}$) in seawater, or can be added in amounts sufficient to support good algal growth. At high pH (pH > 8.0), especially divalent ions have the tendency to precipitate, with phosphate or hydroxide as insoluble salts. For example, the binding capacity of calcium to phosphate is pH dependent, increasing with elevated pH values. Also magnesium precipitates at higher pH, although at a lesser degree than calcium. Compounds which precipitate are no longer available for algal growth. For this reason, the concentrations of ions such as $Ca^{2+}$, $Mg^{2+}$ and phosphate ($PO_4^{3-}$) should be kept as low as possible without limiting (micro-) algae growth.

In a preferred embodiment of the method according to the invention, the cultivating medium comprises Ca and Mg ions, wherein the concentration of Ca ions is between 0.001 and 0.15 mM and the ratio Ca:Mg is between 1:5 and 1:30, preferably 1:10 to 1:20, more preferably 1:15.

Experiments confirm that the amount of magnesium needed by algae is higher than the amount of calcium. Good results are obtained with a ratio of 1:15.

Preferably, Mg ions are provided in a concentration of 0.15 mM and Ca ions in a concentration of 0.01 mM. For example, the medium comprises 0.15 mM $MgCl_2.6H_2O$ and 0.01 mM $CaCl_2.2H_2O$. Preferably the medium further comprises 4.4 mM $KNO_3$ and 0.32 mM polyphosphate. Preferably the medium is based on an marine medium, for example an f/2 medium.

In a preferred embodiment, the method according to the invention further comprises measuring the pH and/or the ion concentration.

Preferably the concentration of magnesium, calcium and/or phosphate is measured. Preferably, the method further comprises adding magnesium, calcium and/or phosphate when the respective measured concentration is below a certain threshold. This has the advantage that the concentration of the ions is kept constant. Additionally or alternatively, the pH level is monitored and the pH is adjusted when it is above or below a given threshold. Preferably the measurement of the ion concentration and/or pH level is performed using one or more sensors which are connected to a control unit, for performing the monitoring and regulation of ion concentrations and/or pH level automatically.

Preferably the cultivation medium comprises Ca and Mg ions, the concentration of Ca ions being between 0.001 and 0.15 mM and the ratio Ca:Mg being between 1:5 and 1:30, preferably 1:10 and 1:20, more preferably 1:15.

Preferably the pH of the medium lies between 9 and 11.

Preferably, bicarbonate and carbonate is provided by $NaHCO_3$ and $Na_2CO_3$. For example bicarbonate and carbonate are present in a concentration of at least 30, 60, 90, 150, 210, 240 or at least 270 mM.

Preferably, the medium comprises NaCl in a concentration in the range 200 to 350 mM. The concentration of the alkali metal chlorine salt in the medium is at least 200 mM, advantageously at least 270 mM, yet more advantageously at least 350 mM, most advantageously at least 400 mM. The alkali metal chlorine salt is advantageously NaCl.

In a preferred embodiment according to the invention, the medium comprises:

- 200-350 mM, preferably 270 mM NaCl;
- 50-100 mM, preferably 75 mM $NaHCO_2$; and
- 50-100 mM, preferably 75 mM $Na_2CO_3$.

This particular composition of the medium surprisingly turns out to enable sufficient growth of algae, while at the same time a low level of precipitation is achieved. Furthermore, the medium enables efficient carbon dioxide transfer.

Preferably the medium further comprises 0.005-0.02 mM Ca and 0.075-0.3 mM Mg and having a pH of 9.0-10.5.

In a preferred embodiment, the medium further comprises one or more suitable vitamins, in particular thiamine, biotin and/or vitamin B12.

In a preferred embodiment, the medium further comprises one or more suitable micronutrients, in particular manganese, zinc, copper, molybdenum and/or cobalt.

In a preferred embodiment, the medium further comprises one or more suitable macronutrients, in particular potassium, phosphorus and/or nitrogen.

The goal of the present invention achieved accordingly, to provide a method and a system for obtaining algal biomass by enhancing the effect of the cultivation pH on the maximal biomass yield and lipid productivity of *N. oleo-*

*abundans* during light-limited growth, specifically by maximizing the relation between the optimal values of obtained biomass density, productivity and yield on light energy at two different pH levels and at a fixed incident light intensity. In particular, without nutrient limitation, the maximal volumetric biomass productivity is approximately two-fold higher under reference conditions than under elevated pH and nitrogen depletion associated with high pH showed to be far more effective in promoting fatty acid accumulation than nitrogen depletion at reference pH. These results indicate the potential of producing high amounts of lipids by cultivating *N. oleoabundans* under the alkaline-saline conditions described in the present invention, possibly integrated in a two-stage process during which algal biomass is first grown under optimal conditions.

Further advantages, features and details of the invention are elucidated on basis of preferred embodiments thereof described in the Examples, wherein reference is made to the accompanying drawings, wherein:

- figure 1 shows a schematic representation of dilution steps in 24-well microtiter plates;
- figure 2 shows a schematic representation of photobioreactor FMT150. Elements 4, 6, 8, 10, 12, 14, 18, 64, 66, 68 correspond to optional connections for operating in turbidostat mode;
- figures 3A-B show the optical density at 750 nm (OD750) of (A) modified f/2 medium at different pH values where the increase in OD750 is a measure of medium precipitation and (B) *N. oleoabundans* in the modified f/2 medium at different pH values as a measure of growth. Lowest and highest pH values tested are represented by white and black triangles, respectively. Error bars represent the standard deviation from the average of the replicates;
- figures 4A-B show the growth of *N. oleoabundans* in dilution series at (A) pH 8.1 and (B) pH 10.0. Growth rates determined 24 hours after each dilution step;
- figures 5A-B show the growth of *N. oleoabundans* in turbidostat cultivation at (A) pH 8.1 and (B) pH 10.0. The vertical dotted lines represent the point after what depletion of nitrogen from the medium was applied; and
- figures 6A-D show pictures taken during the turbidostat experiment at pH 10.0. (A) When in N-replete medium, most part of the cells showed red fluorescence emitted by the chloroplasts. (B), (C) and (D) Green fluorescence emitted by lipid bodies in nitrogen starved cells. All images are 1000 times magnified.
- Figure 7A-B shows the influence of dilution rate (D, d-1) on (A) biomass concentration (Cx, $g_{DW} L_{-1}$) and (B) biomass productivity (Px,vol, $g_{DW} L_{-1} d_{-1}$) of N. *oleoabundans* at reference conditions (pH 8.2, closed symbols) and at higher pH (pH 10.0, open symbols). Values for maximal Px,vol and corresponding optimal Cx are indicated by rectangles. Error bars represent the standard deviation from the average of the replicates.
- Figure 8 show the influence of dilution rate (D, $d_{-1}$) on the biomass yield on light energy ($Y_{x/E}$, $g_{DW} mol_{ph-1}$) of N. *oleoabundans* at reference conditions (pH 8.2, closed symbols) and at higher pH (pH 10.0, open symbols). Error bars represent the standard deviation from the average of the replicates.
- Figure 9 show the ifluence of growth rate ($\mu$=D, $d_{-1}$) on the specific light absorption rate ($r_{E/x}$, $mol_{ph} g_{DW} d_{-1}$) of *N. oleoabundans* at reference conditions (pH 8.2, closed symbols) and at higher pH (pH 10.0, open symbols). (A) takes into account all data points measured during the experiments and (B) assumes that oversaturation takes place at D=1.9 $d_{-1}$ and excludes the correspondent data points. Error bars represent the standard deviation from the average of the replicates.
- Figure 10 show fatty acid composition (% w/w) of N. *oleoabundans* at the highest dilution rate applied (D=1.9 $d_{-1}$). Results presented for reference conditions (pH 8.2) and for higher pH (pH 10.0) experiments, without and with nitrogen depletion (N(+) and N(-), respectively).
- Figure 11 - Pictures taken during chemostat experiments at pH 10.0 after cell staining with Bodipy 505/515. (A) Red chlorophyll autofluorescence under N-replete conditions.

## Examples

### Example 1: Materials and Methods

#### *Microalgal strain*

Experiments were performed in a lab-scale photobioreactor, and in microtiter plates to determine the specific growth rate of microalga of the species *Neochloris oleoabundans* under a range of alkalinities and salinities. Besides nutrient-replete growth, nitrogen deprivation was applied to induce lipid accumulation and to check whether this accumulation was affected by the alkalinity and the salinity of the growth medium. Moreover, the development of a stable medium under those conditions is discussed.

*N. oleoabundans* UTEX 1185 was obtained from the algae culture collection of the University of Texas, Austin, USA and pre-cultivated in 250 mL shake flasks containing 100 mL of growth medium. We placed them on a orbital shaker incubator (Innova 44, New Brunswick Scientific, New Jersey, USA) under a 2% $CO_2$ enriched headspace and a light intensity of 30 $\mu$mol photons $m^{-2}s^{-1}$ provided by fluorescent lamps. Temperature was controlled at 25 °C and the pH was adjusted to 7.8.

### Medium design and microalgal growth

A modified f/2 medium was used for growth as described in table 1.

*Table 1: Modified f/2 medium composition. X and Y are the concentrations of magnesium and calcium in artificial seawater, respectively. Values for these concentrations were determined in the experiments, as described below.*

| Compound | [mM] |
| --- | --- |
| **Macronutrients** | |
| $KNO_3$ | 4.4 |
| Super FK ($P_2O_5$) | 0.32 |
| EDTA ferric sodium salt | 29.28 $\mu M$ |
| **Vitamins** | |
| Thiamine | 200 $\mu$ L$^{-1}$ |
| Biotin | 1 $\mu$ L$^{-1}$ |
| $B_{12}$ | 1 $\mu$ L$^{-1}$ |
| **Micronutrients** | |
| EDTA-MnNa$_2$ | 1.5 E-02 |
| EDTA-ZnNa$_2$ | 2.0 E-03 |
| EDTA-CuNa$_2$ | 7.0 E-03 |
| $Na_2MoO_4.2H_2O$ | 7.7 E-04 |
| $CoCl_2.6H_2O$ | 3.7 E-04 |
| **Artificial seawater** | |
| NaCl | 420.0 |
| $MgCl_2.6H_2O$ | *X* |
| $CaCl_2.2H_2O$ | *Y* |
| $Na_2SO_4$ | 22.53 |

Non-biological precipitation experiments were performed in 24-well microtiter plates (Falcon 3047, BD Biosciences, California, USA), by manipulating the cultivation medium composition in order to test its stability at elevated pH. The plates were fixed inside the orbital shaker incubator rotated at 150 rpm with an orbit of 2.54 cm. Wells with demineralised water were taken as blanks for the measurement of optical density (OD). In a total volume of 1.5 mL per well, different $Mg^{2+}/Ca^{2+}$ combinations in f/2 medium were tested. The ratio between both elements was either kept constant (1:13), or varied with a constant minimal calcium concentration (0.01 mM). Phosphate and nitrate were kept in a ratio of 1:14 at levels sufficient to sustain 1 g L$^{-1}$ of biomass. The phosphate source in this modified medium was a potassium polyphosphate solution insensitive to high pH levels (Super FK, YARA, Vlaardingen, The Netherlands). Six different pH values were tested in duplicate for each combination: 9.0, 9.5, 10.0, 10.3, 10.5 and 10.8. The desired pH was obtained by buffering the wells with mixtures of sodium bicarbonate ($NaHCO_3$) and disodium carbonate ($Na_2CO_3$) in a total concentration of 300 mM, as described in table 2.

*Table 2: Molar concentrations of NaHCO3 and Na2CO3 in the buffering mixtures, for each pH tested.*

| pH | $NaHCO_3$ [mM] | $Na_2CO_3$ [mM] |
| --- | --- | --- |
| 9.0 | 270 | 30 |
| 9.5 | 210 | 90 |
| 10.0 | 150 | 150 |
| 10.3 | 90 | 210 |
| 10.5 | 60 | 240 |
| 10.8 | 30 | 270 |

The stability of the medium was assessed by following the optical density at 750 nm (OD750) in time using a multilabel plate reader (VICTORTM X3, PerkinElmer, Massachusetts, USA). The light scattered by the insoluble particles (i.e. salt crystals) in suspension determines the optical density at 750 nm and can be used as a measure of the formation of those particles.

The growth of *N. oleoabundans* was studied in this modified medium, under the same conditions, except for the supply of light and carbon dioxide. Light was supplied by fluorescent light tubes at an intensity of 30 $\mu$mol photons $m^{-2}s^{-1}$ and $CO_2$ by applying a headspace with 4% $CO_2$-enriched air.

In these experiments, 75 $\mu$L from 2 weeks old shake flask cultures were used as inoculum in a total of 1.5 mL per well. Wells filled with solely medium were used as controls. The optical density at 750 nm was measured daily to follow biomass growth.

### *Growth assessments in 24-well microtiter plates*

The influence of a range of alkalinities and salinities on growth was also studied in 24-well microtiter plates. The wells were filled with 1.350 mL of the modified f/2 medium and 150 $\mu$L of inoculum from 2 weeks old shake flasks cultures. Wells filled with solely 1.5 mL of medium were used as blanks for the measurement of optical density (OD). The plates were placed in a chamber fixed inside the orbital shaker incubator, rotated at 150 rpm with an orbit of 2.54 cm. Continuous light was supplied by fluorescent lamps at an intensity of 55 $\mu$mol photons $m^{-2}s^{-1}$. The chamber's headspace was continuously flushed with humidified and $CO_2$-enriched air. Both gas flows were set by mass flow controllers (Bronkhorst Nederland B.V., Veenendaal, The Netherlands). Microalgae growth was assessed at 30°C and four pH values: 8.1, 9.0, 10.0 and 10.5. The pH was set by buffering the growth medium with mixtures of $NaHCO_3$ and $Na_2CO_3$, in total concentrations of 300 mM and 150 mM (table 3) and adjusting the $CO_2$ fraction in the headspace.

*Table 3: Molar concentrations of NaHCO3 and Na2CO3 in the buffering mixtures used in total concentrations of 300 and 150, for each pH tested.*

| Total [mM] | 300 | | 150 | |
|---|---|---|---|---|
| pH | NaHCO$_3$ [mM] | Na$_2$CO$_3$ [mM] | NaHCO$_3$ [mM] | Na$_2$CO$_3$ [mM] |
| 9.0 | 270 | 30 | 135 | 15 |
| 10.0 | 150 | 150 | 75 | 75 |
| 10.5 | 60 | 240 | 30 | 120 |

By varying the concentration of sodium chloride (NaCl), also the algae's salt tolerance was checked. In four sets of experiments, different concentrations of NaCl in the medium were combined with the $NaHCO_3$ and $Na_2CO_3$ mixtures (table 4).

*Table 4: Sodium chloride combinations with the NaHCO$_3$ and Na$_2$CO$_3$ buffering mixtures, used in each set of experiments.*

| Se t | NaCl [mM] | NaHCO$_3$ + Na$_2$CO$_3$ [mM] |
|---|---|---|
| 1 | 420 | 300 |
| 2 | - | 300 |
| 3 | 120 | 300 |
| 4 | 270 | 150 |

This was done at each pH value, except for the reference at pH 8.1, where only f/2 medium with 12 mM $NaHCO_3$ was used. All sets of experiments were performed in four replicates to check reproducibility.

Optical density at 750 nm was measured daily using a multilabel plate reader (VICTORTM X3, PerkinElmer, Massachusetts, USA). After 24 hours of inoculation, the suspensions with known initial volumes (Vs) and OD750 ($OD_{start}$) were diluted with a certain volume of medium (Vm) in such a way that after another 24 hours of growth the same OD750 ($OD_{end}$) was reached. Specific growth rates ($\mu$) were then calculated for all the replicates according to equation A:

$$\text{(equation A)} \quad \mu = \frac{\ln\left\{OD_{end}\bigg/\left[\dfrac{OD_{start} \times V_s}{V_s + V_m}\right]\right\}}{24}$$

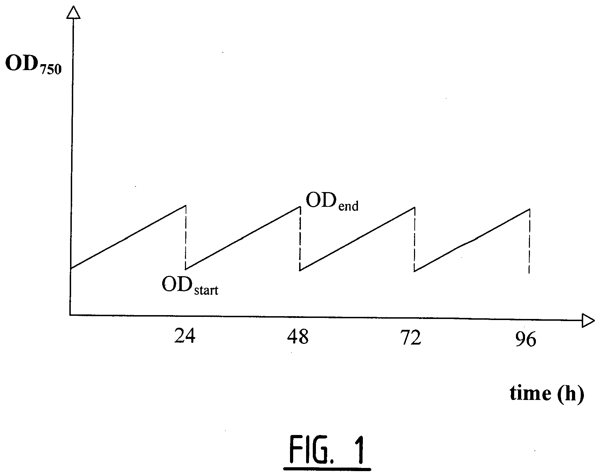

The average $\mu$ and corresponding standard deviations were determined and used to estimate growth. The dilution series were done every 24 hours, until a constant specific growth rate was observed, as schematically represented in figure 1, after which the cells were allowed to grow until the stationary phase.

**Growth assessments in a flat panel photobioreactor** *Photobioreactor set-up*

The microalgae were also grown in a flat panel photobioreactor (FMT150, Photon Systems Instruments, Brno, Czech Republic) with a detachable rectangular cuvette 20 cm high, 10 cm wide and 2.5 cm of light path.

Medium container 4 is connected via filter 6 to outlet 8 and via line 10 with peristaltic pump 12. Pump 12 is connected via line 14 with medium inlet 16. Via line 17 the medium is directed towards the bottom of reactor 2. Pump 12 is connected via control signal line 18 to control unit 20, which is connected via data visualisation line 22 and data collection line 24 to local computer processing unit 26. Processing unit 26 is optionally connected to remote PC 30 via line 28.

Photobioreactor 2 comprises an optional sensor 32 for instance for measuring optical density. Preferably the sensor is connected to control unit 20.

Air inlet 34 and $CO_2$ inlet 36 are connected via mass flow controllers 38 and line 40 to bubble interruption valve 42, which is connected via lines 44 to gas humidification bottle 46. Line 48 connects the gas humidification bottle 46 via filter 50 and line 52 to gas inlet 54 of the reactor 2. Gas inlet 54 is connected via line 56 to sparger 57 which releases the gas at the bottom of reactor 2.

Reactor 2 further comprises a pH sensor 58, which is preferably connected to control unit 20. Furthermore, a sample outlet 60 and a gas/liquid outlet 62 are provided. Gas/liquid outlet 62 is connected via line 64 to overflow unit 66 which is connected to filter 68.

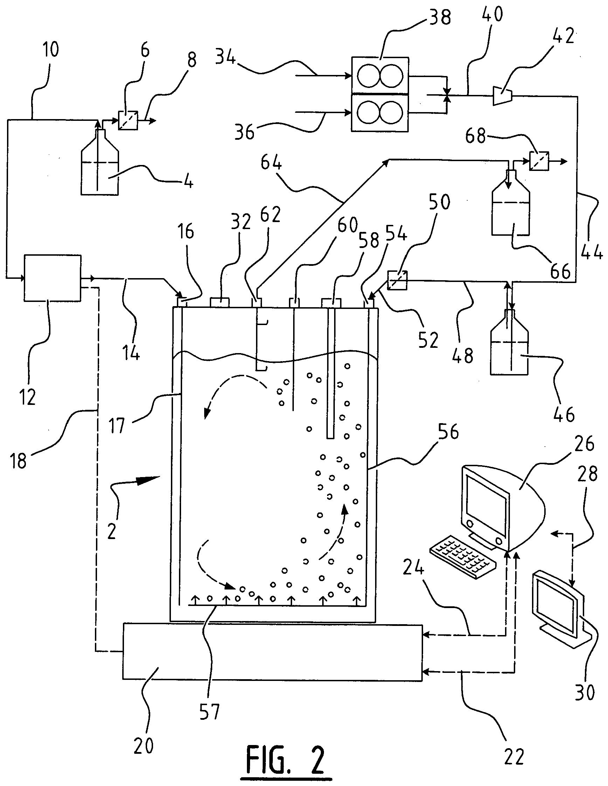

The photobioreactor (PBR) 2 (figure 2) was equipped with temperature control and a light source composed of red emitting diodes, LEDs ($\lambda$max = 627 nm). A built-in dual-wavelength densitometer 32 allowed us to monitor the culture growth and the chlorophyll absorption through the integrated measurement of optical density at 735 nm and 680 nm, using the PBR itself as cuvette (2.5 cm optical path). In turbidostat mode the PBR also controlled a peristaltic pump 12 in order to maintain a constant OD735 by the addition of fresh medium and concomitant harvesting of surplus culture.

The incident light flux was measured at 15 different places equally distributed over the PBR's light-exposed surface with a LI250 PAR 2n quantum sensor (Li-COR®, Lincoln, USA). The average light at the surface was set to 300 $\mu$mol photons m$^{-2}$s$^{-1}$ and the temperature was maintained at 30 °C. Mixing was performed by continuous sparging at the bottom of the cuvette with a filtered $CO_2$-enriched air stream, saturated with water before entering the vessel. The flows of air and $CO_2$ were set by mass flow controllers (Bronkhorst Nederland B.V., Veenendaal, The Netherlands) to a total of 650 ml min$^{-1}$. The flow of $CO_2$ in the gas stream was adjusted according to the pH required in the study.

***Microalgae growth in turbidostat cultivation and lipid accumulation under nitrogen depletion***

The influence of the pH on growth was studied in turbidostat mode at pH 8.1 and pH 10.0. At both pH the algae were deprived of nitrogen at the end of the experiments to induce the accumulation of lipids (oil bodies). The concentration of NaCl in the medium was 419 mM at pH 8.1 and 270 mM at pH 10.0. The pH was determined by the $CO_2$ supplied through the gas stream and by the addition of $NaHCO_3$ and $Na_2CO_3$ to the medium. At pH 8.1, 12 mM $NaHCO_3$ was in equilibrium with 1% v/v $CO_2$. At pH 10.0, a mixture of 75 mM $NaHCO_3$ and 75 mM $Na_2CO_3$ was necessary while gassing with only 0.1% v/v $CO_2$.

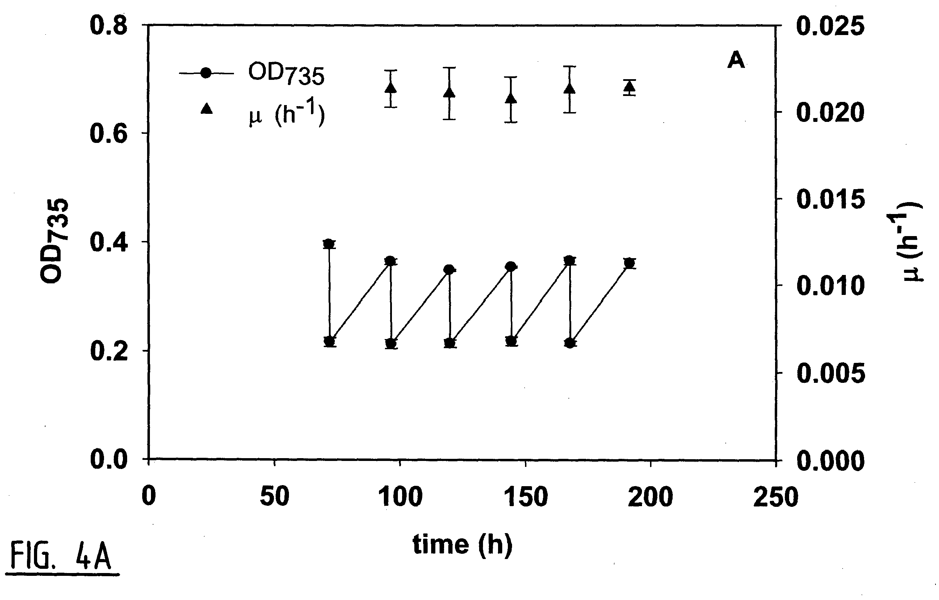

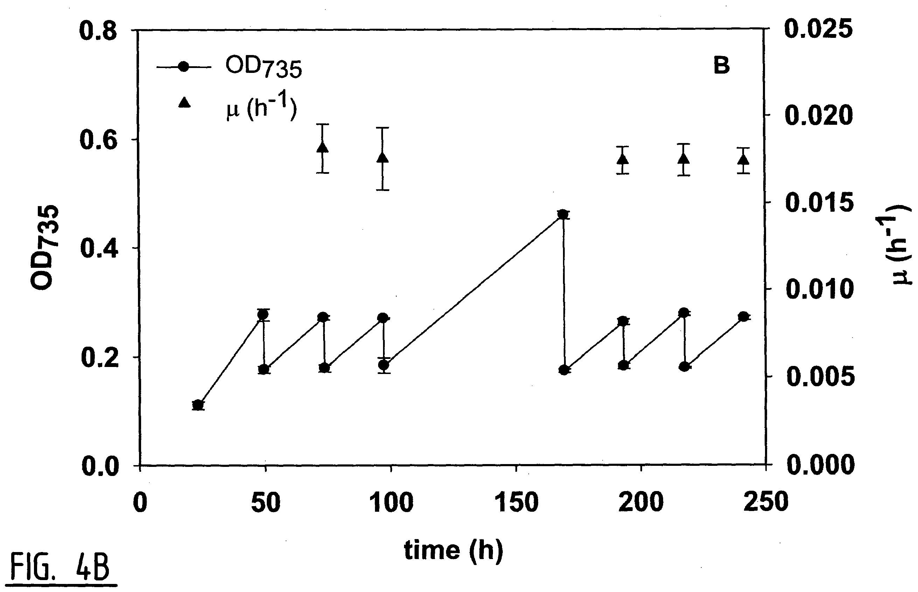

The working volume of the photobioreactor (figure 2) was 385 mL composed of 355 mL of filtered medium and 30 mL inoculum from a 2-week old shake flask culture. Growth started batch wise in the modified f/2 medium (table 1) with optimal $Ca^{2+}$ and $Mg^{2+}$ salt concentrations (X and Y, see results) and the amounts of $NaHCO_3$ and $Na_2CO_3$ given above. An OD735 of 0.2 was defined as the set-point (e.g. figure 4). At this point the turbidostat mode was activated and the suspension's optical density was kept constant, by dilution with fresh medium each time values above 0.2 were measured. This allowed determining $\mu$ under steady-state, which was equivalent to the dilution rate (D). Additionally, the correspondent biomass concentrations (Cx) were determined by dry weight measurements.

Nitrogen depletion was applied when the growth rate was constant in nitrogen-replete conditions, by replacing the medium with N-free medium resulting in a gradual but complete depletion of nitrogen.

***Biomass determination***

Triplicate samples of ten grams of the algae suspension were filtrated over pre-dried and pre-weighted Whatman GF/F glass fiber filters (Ø 55 mm, nominal pore size 0.7 $\mu$m). The wet filters were then dried at 95 °C for 24 hours, placed in a dessicator to cool down for 2 additional hours and weighted again. The dry weight was determined as the difference between the weights of the dried filters containing the samples and the dried empty filters.

*Visualization of intracellular neutral lipid bodies*

The accumulation of intracellular lipid bodies by the algae was checked by fluorescence microscopy. The lipophilic fluorescent dye BODIPY 505/515 (4,4-difluoro-1,3,5,7-tetramethyl-4-bora-3a,4a-diaza-sindacene; Invitrogen Molecular Probes, Carlsbad, CA) was used for staining the cells, according to Cooper et al. (2010). A 40 $\mu$M BODIPY 505/515 stock solution was prepared by dissolving the dye in 0.2% (v/v) of anhydrous dimethyl sulfoxide (DMSO), used as a vehicle to facilitate the permeation of the dye into the cells. Aliquots of this stock solution were added directly to the algal cell suspensions in order to have a final dye concentration of 1 $\mu$g mL-1 and 0.02% DMSO. After 5 minutes of contact at 25 °C, in the dark with agitation, slides were prepared for observation under an inverted fluorescent microscope (Leica DMI6000, Leica Microsystems B.V., Rijswijk, The Netherlands). Excitation was done under a blue excitation light through a band-pass filter at 450-490 nm. Emission wavelengths were imaged through a long-pass filter of 515 nm. BODIPY 505/515 has a green emission spectrum which makes the stained lipid bodies distinct from the endogenous red autofluorescence emitted by the chloroplasts under the same blue excitation light.

*Fatty acids quantification*

Duplicate samples of the algae suspension with a known volume were centrifuged and the wet pellets were frozen and stored at -80°C. Before the fatty acids extraction itself, those pellets were resuspended and transferred to bead beating tubes. Those tubes were lyophilized overnight and the exact amount of biomass was determined. 1 mL of a 2:2.5 mixture of chloroform and methanol containing 42 $\mu$g mL-1 of Tripentadecanoin (C15:0 TAG, Sigma-Aldrich T4257, as internal standard) was used to dissolve the dried biomass. To disrupt the cells, the bead beating tubes were placed in a bead beater for 40 minutes. The liquid samples were then transferred to 10 mL Pyrex tubes with Teflon coated screw caps. Additional 3 mL of the same mixture were used to rinse the bead beating tubes to recover the whole biomass. After proper homogenization, the tubes were placed in a water bath for sonication during 10 min. 2.5 mL of 50 mM Tris-buffer (containing 1M NaCl, pH = 7.5) were added to separate the polar from the apolar phase. The tubes were then centrifuged for 5 minutes at 2500 rpm and 15 °C. The phase separation bottom layers (first chloroform fractions) were transferred to new tubes while cell debris and the polar phases were reextracted twice with 1 mL of chloroform. The separation of the pooled chloroform from the algal material was done by evaporation under pure nitrogen for around 45 minutes (or until all the chloroform was gone). 3 mL of 5% (v/v) sulfuric acid in methanol was added to the tubes to convert the fatty acids into fatty acid methyl esters (FAMEs). The samples were then incubated in a block heater for 3 hours at 70°C. After cooling down the samples to room temperature, 3 mL of hexane and 3 mL of demineralized water were added and the tubes were centrifuged for 5 minutes at 2500 rpm and 15 °C. Aliquots of around 2 mL where collected from the phase separation upper layer (hexane and fatty acids) to new tubes. 2 mL of demineralized water were added to wash the hexane during a last step of centrifugation. The hexane phases containing the fatty acids were analyzed by gas chromatography (GC) using a HP 6890 (Hewlett Packard Inc., Supelco NucolTM col. 355 33-03A, 30m x 530$\mu$m x 1.0$\mu$m). The FAMEs were identified by comparison of their retention times with those of standard fatty acids (Sigma Aldrich, MO, USA).

Quantification of the identified lipids was done according to the biomass dry weight and by comparing their respective peak areas. Amounts were corrected for the response factors of each fatty acid.

**Results and Discussion**

*Growth of N. oleoabundans in a modified f/2 medium*

The preparation of cultivation media with high concentrations of salts and high pH needs careful attention regarding the issue of precipitation. Compounds which precipitate are no longer available for microalgal growth. For instance, the binding capacity of calcium to phosphate is pH dependent, increasing at elevated values.

A chemical equilibrium model for the calculation of saturation indices in aqueous solutions was the base for developing a medium with low levels of precipitation. A simulation showed for example high formation of a calcium phosphate complex, hydroxyapatite, for our modified f/2 medium with a pH of 10.5 containing $Ca^{2+}$ and $PO_4^{3-}$ in concentrations of 3.61 mM and 5 mM, respectively. One way to reduce the presence of hydroxyapatite is to decrease the concentrations of $Ca^{2+}$ and/or $PO_4^{3-}$.

The elemental composition of Chlorella vulgaris, was used as a basis for determining the concentrations of nitrogen and phosphate necessary to produce 1 g L-1 of biomass, defined to be sufficient for this study. It was determined that 4.4 mM $NO_3^{-}$ and 0.32 mM $PO_4^{3-}$ need to be present in solution to reach such biomass concentration.

Non-biological experiments showed that $Ca^{2+}$ in a concentration of 0.01 mM leads to a medium with very low complexation levels at high pH values. Besides calcium, also magnesium can complex and precipitate at high pH, though at a lesser degree. Moreover, the amount of magnesium needed by the cells seems to be higher than calcium. The best ratio we obtained between $Ca^{2+}$ and $Mg^{2+}$ was 1:15, the latter being in a concentration of 0.15 mM.

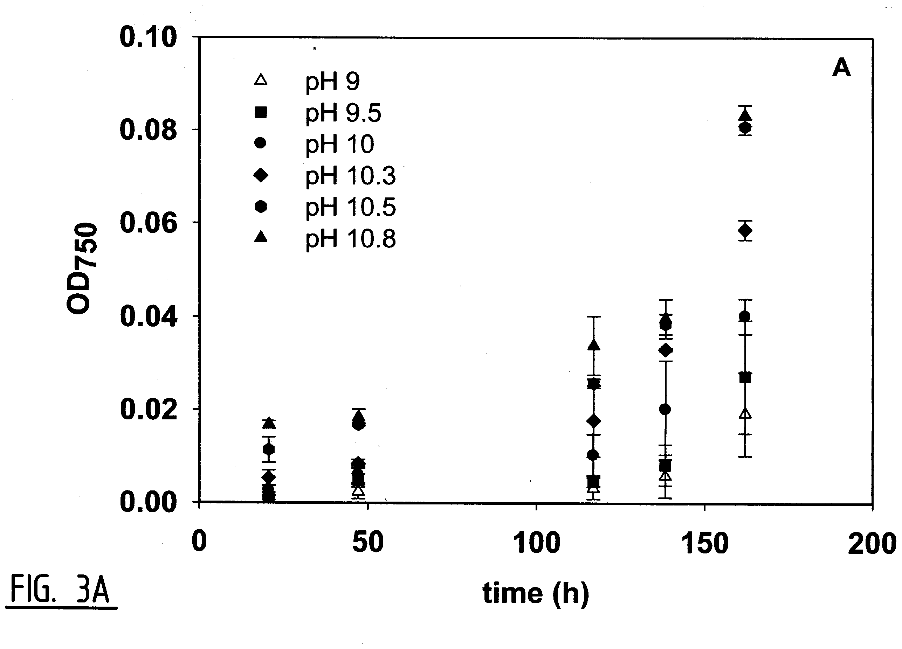

According to the present invention, the medium is based on f/2 medium with 0.01 mM $CaCl_2$ (X), 0.15 mM $MgCl_2$ (Y), 4.4 mM $KNO_3$ and 0.32 mM 'P' (from the polyphosphate). In figure 3A the optical density shows the level of precipitation at different pH values. In the first 100 hours the OD hardly increases. Only at elevated pH (>10) and after extended time (150 hours) limited precipitation occurs. For continuous microalgae production this should not cause any problems since the rate of consumption will be faster than the rate of precipitation. Moreover, continuous consumption will result in even lower concentrations of $Ca^{2+}$, $Mg^{2+}$ and $PO_4^{3-}$, further reducing the risk of precipitation.

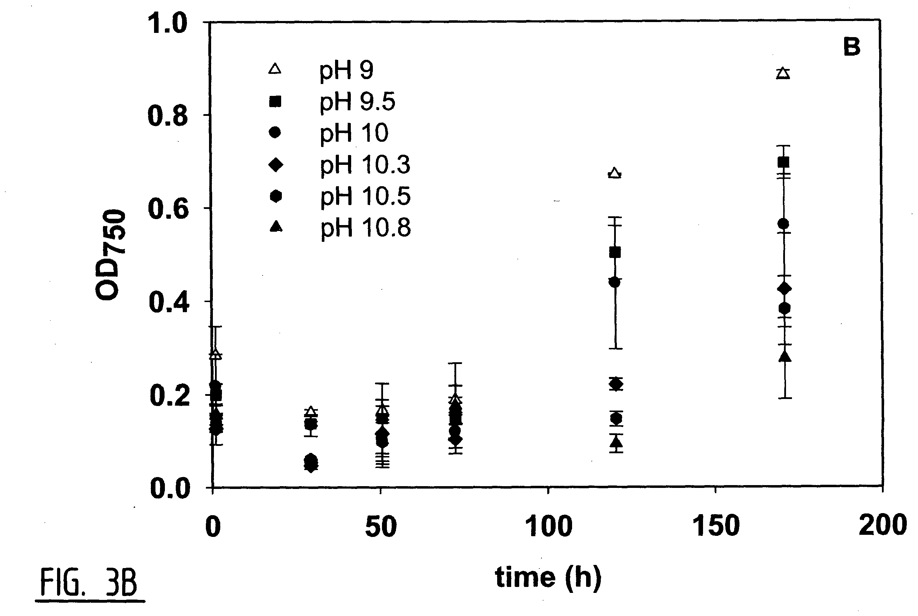

When cultivating *N. oleoabundans* in the modified medium a decrease in growth with the increased pH was observed (figure 3B). However, growth was apparently not limited by the low concentrations of $Ca^{2+}$ and $Mg^{2+}$ and the cells could reach a maximal OD750 of about 0.9 at pH 9.0 and of almost 0.6 at pH 10.0.

The values of the graphs of figure 3 are listed below in table 5A-B.

*Table 5A: Optical density at 750 nm ($OD_{750}$) of modified medium at different pH values where the increase in OD750 is a measure of medium precipitation.*

| | pH 9 | | pH 9.5 | | pH 10 | |
|---|---|---|---|---|---|---|
| time (h) | OD750 | std error | OD750 | std error | OD750 | std error |
| 20.518 | 1.83E-03 | 3.17E-04 | 1.04E-03 | 4.24E-04 | 2.65E-03 | 1.05E-03 |
| 47.085 | 2.37E-03 | 1.47E-03 | 4.81E-03 | 1.49E-03 | 6.11E-03 | 1.87E-03 |
| 116.96 | 3.19E-03 | 2.29E-03 | 4.66E-03 | 1.17E-03 | 0.0104 | 4.39E-03 |
| 138.37 | 5.85E-03 | 4.66E-03 | 8.24E-03 | 4.49E-03 | 0.02 | 0.0105 |
| 161.95 | 0.0191 | 8.86E-03 | 0.0271 | 0.0121 | 0.04 | 3.78E-03 |

| | pH 10.3 | | pH 10.5 | | pH 10.8 | |
|---|---|---|---|---|---|---|
| time (h) | OD750 | std error | OD750 | std error | OD750 | std error |
| 20.5175 | 5.36E-03 | 1.72E-03 | 0.0114 | 2.71E-03 | 0.0168 | 8.20E-04 |
| 47.0847 | 8.43E-03 | 8.96E-04 | 0.0168 | 3.22E-04 | 0.0184 | 1.69E-03 |
| 116.955 | 0.0176 | 7.60E-03 | 0.0256 | 9.60E-04 | 0.0337 | 6.30E-03 |
| 138.367 | 0.0329 | 3.00E-04 | 0.0383 | 2.21E-03 | 0.0395 | 4.20E-03 |
| 161.952 | 0.0585 | 2.13E-03 | 0.0808 | 1.68E-03 | 0.0832 | 2.24E-03 |

*Table 5B: Optical density at 750 nm (OD750) of N. oleoabundans in the modified medium at different pH values as a measure of growth.*

| (B) | pH 9 | | pH 9.5 | | pH 10 | |
|---|---|---|---|---|---|---|
| time (h) | OD750 | std error | OD750 | std error | OD750 | std error |
| 1.0897 | 0.2838 | 0.0622 | 0.1993 | 0.0228 | 0.2172 | 0.0689 |
| 29.259 | 0.1607 | 6.90E-03 | 0.1374 | 4.64E-03 | 0.134 | 0.0231 |
| 50.595 | 0.1653 | 9.27E-03 | 0.1478 | 0.0746 | 0.1056 | 0.0487 |
| 72.383 | 0.1875 | 5.52E-03 | 0.1485 | 0.0126 | 0.1202 | 0.0353 |
| 120.38 | 0.6704 | 1.95E-03 | 0.5025 | 0.0572 | 0.4364 | 0.141 |
| 170.9 | 0. 883 | 9.03E-03 | 0.6942 | 0.0346 | 0.5597 | 0.1095 |

| (B) | pH 10.3 | | pH 10.5 | | pH 10.8 | |
|---|---|---|---|---|---|---|
| time (h) | OD750 | std error | OD750 | std error | OD750 | std error |
| 1.0897 | 0.1265 | 0.0338 | 0.1378 | 0.018 | 0.1576 | 0.0226 |
| 29.2589 | 0.0472 | 6.66E-03 | 0.0608 | 9.24E-04 | 0.0537 | 4.29E-03 |
| 50.5953 | 0.1162 | 0.0721 | 0.0981 | 0.0468 | 0.1027 | 0.0359 |
| 72.3825 | 0.1037 | 0.0307 | 0.1624 | 0.0556 | 0.1756 | 0.0904 |
| 120.38 | 0.2197 | 0.0122 | 0.146 | 0.0156 | 0.0941 | 0.0198 |
| 170.895 | 0.4223 | 0.1202 | 0.3798 | 0.0391 | 0.274 | 0.0862 |

**Example 2.** *Influence of pH and salt concentration on specific growth rate*

*Microalgae growth in 24-well microtiter plates*

The methods for producing the microalgal strain and growth medium, as well as the photobioreactor are such as described in Example 1.

For all the pH values tested, no growth was observed with Set 1 (highest total salt concentration), suggesting that *N. oleoabundans* cannot stand such saline circumstances. At higher pH levels with less $HCO_3^-$/$CO_3^{2-}$ (total 150 mM), the combination with more NaCl (270 mM/Set 4) seems to be better for the algae than less NaCl (absent/Set 2 and 120 mM/Set 3) combined with more $HCO_3^-$/$CO_3^{2-}$ (total 300 mM). A medium with 270 mM NaCl and 150 mM $HCO_3^-$/$CO_3^{2-}$ is therefore at this moment considered the optimal formulation capable of supporting growth at pH 10.0. Very poor growth was observed at pH 10.5, for all sets of experiments. Growth rates were not determined for this value.

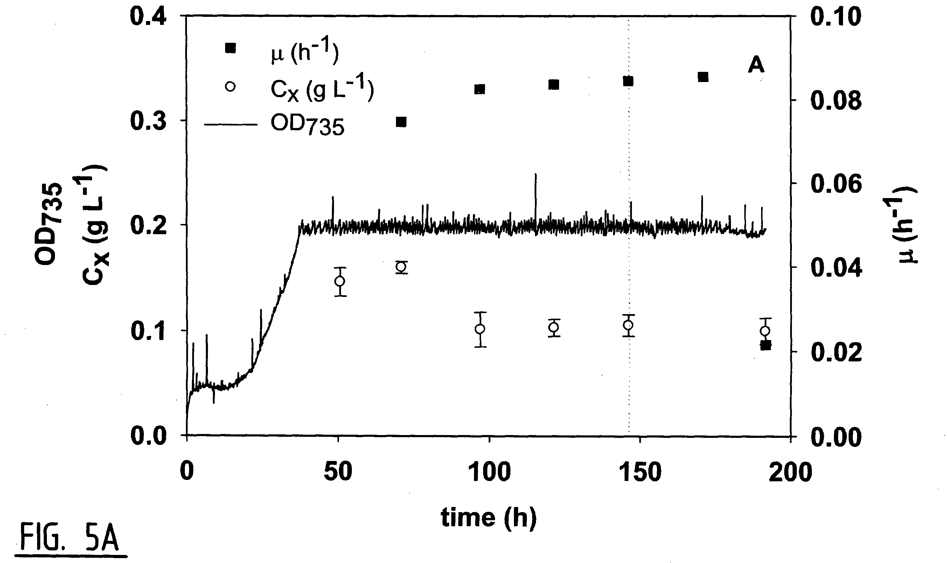

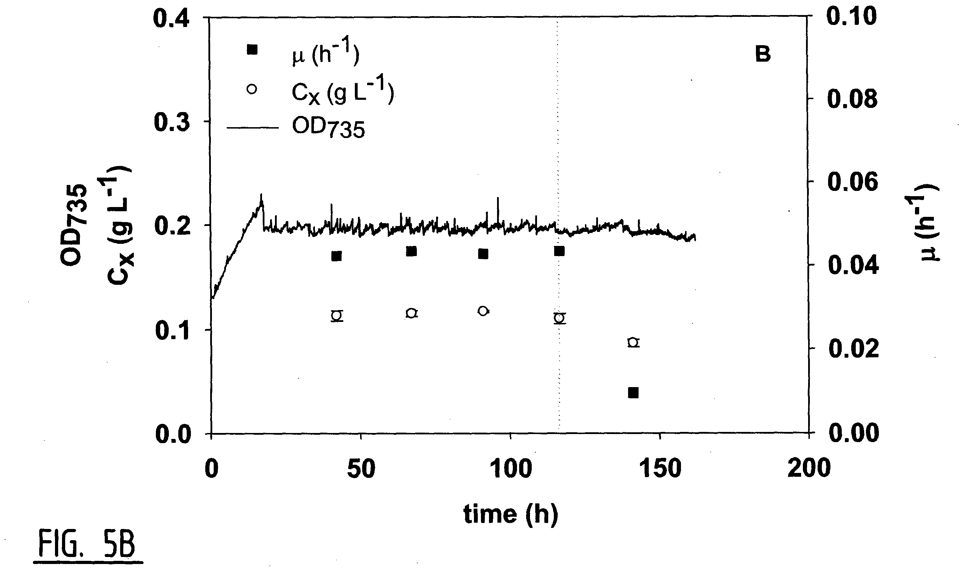

An average growth rate of 0.0212 h$^{-1}$ was found for the reference experiment at pH 8.1 (figure 5A), being the highest amongst all the experiments. However, this value was not considerably different for the ones found at pH 10.0, where the specific growth rate reached 0.0177 h$^{-1}$ (figure 5B).

A correlation between the pH and the salt concentration seems evident and both have an impact on microalgal growth. Although the specific growth rates are all relatively low, due to light limitation inside the incubators ($\sim 55$ µmol photons m$^{-2}$s$^{-1}$), they give a good insight on which should be the best conditions to grow *N. oleoabundans* at high pH: a marine medium with 270 mM NaCl, 75 mM $NaHCO_2$ and 75 mM $Na_2CO_3$.

**Example 3:** *Microalgae growth in turbidostat cultivation and lipid accumulation under nitrogen depletion*

The methods for producing the microalgal strain and growth medium, as well as the photobioreactor are such as described in Example 1.

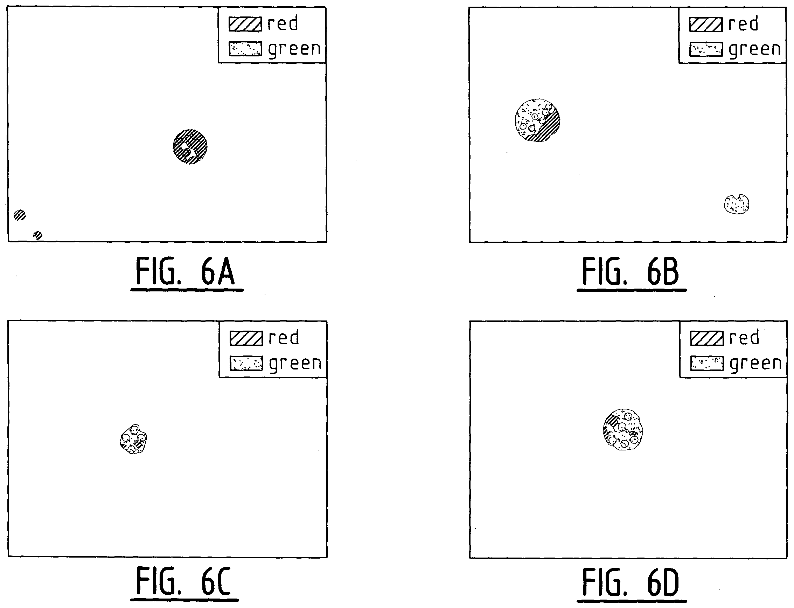

To estimate the growth under high pH and salt concentration under controlled conditions in a continuous system, we performed two turbidostat experiments, at pH 8.1 and pH 10.0. In addition, the light intensity applied was of saturating nature (300 µmol photons m$^{-2}$s$^{-1}$) and allowed to assess the specific growth rate without any light limitation. The total salt concentration was 420 mM according to the study performed in the microtiter plates. The respective biomass concentrations (Cx) and dilution rates (D) were determined under steady-state. In steady state the specific rate of cell growth equals the rate of dilution. Growth rates of 0.08 h$^{-1}$ and 0.04 h$^{-1}$ were found at pH 8.1 and pH 10.0 (figure 6A and 6B, respectively). Cx was constant under steady-state, being about 0.1 g L$^{-1}$ in both experiments. Although the growth rate we obtained at pH 10.0 was twice as small as that at pH 8.1, it is still comparable with previous values obtained in the prior art in freshwater medium (bold basal medium) at pH 7.5 and 25 °C.

Nitrogen starvation is known to be a strong inducer of lipid accumulation. After reaching a constant growth rate, the cultivation medium was replaced by nitrogen-free medium. This resulted on a sharp decrease in µ, to 0.02 h$^{-1}$ at pH 8.1 and 0.01 h$^{-1}$ at pH 10.0. This decrease is obviously caused by the absence of nitrogen in the medium which was already reported to be a growth limiting factor.

Cells growing in the photobioreactor at pH 8.1 and 10.0 were also analyzed for their total fatty acid content and composition, both under nitrogen-replete conditions and after 1 day of nitrogen depletion. Table 6 shows the microalgal content in each of the identified fatty acid, as well as their relative percentage of the total fatty acid pool.

*Table 6: Fatty acid content (% w/w) of Neochloris oleoabundans grown at pH 8.1 and pH 10.0 in turbidostat experiments, without nitrogen limitation (N+) and with nitrogen depletion (N-). The correspondent relative compositions (%rel of the total) are also shown.*

| Fatty acid | Content (% w/w) | | | | | | | |
|---|---|---|---|---|---|---|---|---|
| | pH 8.1 N+ | %rel | pH 8.1 N- | %rel | pH 10.0 N+ | %rel | pH 10.0 N- | %rel |
| C16:0 | 2.41 | 31.1 | 3.92 | 29.6 | 2.74 | 23.8 | 7.79 | 22.3 |
| C16:1 | 0.22 | 2.8 | - | - | 0.31 | 2.7 | 0.14 | 0.4 |
| C18:0 | 0.17 | 2.2 | 0.35 | 2.6 | 0.39 | 3.4 | 0.76 | 2.2 |
| C18:1 | 1.00 | 12.9 | 4.00 | 30.2 | 3.34 | 29.0 | 15.22 | 43.6 |
| C18:2 | 2.14 | 27.6 | 3.08 | 23.3 | 3.23 | 28.0 | 9.25 | 26.5 |
| C18:3 | 1. 80 | 23.6 | 1.88 | 14.2 | 1.51 | 13.1 | 1.72 | 4.9 |
| *Total* | *7. 74* | | *13.23* | | *11.52* | | *34.88* | |

An increase in the total fatty acid content of the nitrogen starved cells was observed, just one day after switching from N-replete medium to N-free medium. This increase was even more pronounced for the cells growing under those conditions at pH 10.0 (up to 35% of dry weight), which may suggest that an additional stress such as high pH could promote lipid accumulation further.

The most abundant fatty acids under nitrogen depletion are the C16:0 and the C18:1. The results also show a relative increase of about two-fold of C18:1 fatty acids under nitrogen deplete conditions for both pH values. Values for microalgal fatty acids content and their accumulation at high pH have never been reported before.

The visualization of the neutral lipid bodies inside the cells was possible using fluorescence microscopy. After a fast diffusion of BODIPY 505/515, the intracellular lipid bodies emitted a strong green fluorescence and could be easily distinguished from the chloroplasts, which appeared in red under the same excitation wavelength. During the course of both experiments the cells showed green fluorescent spherical bodies, either with or without nitrogen in the medium. However, that accumulation was more evident upon nitrogen starvation, with the majority of the cells showing a sharp and strong green fluorescence. Figure 6 shows the accumulation of those bodies in cells growing under pH 10.0.

The growth of the freshwater *N. oleoabundans* was investigated under alkaline-saline conditions. A stable cultivation medium was developed under these conditions based on minimal growth requirements and maximal solubility of nutritious salts. An artificial seawater medium with reduced $Ca^{2+}$ and $PO_4^{3-}$ was successful in decreasing mineral precipitation related with high pH levels. The obtained growth rate in this modified medium with 420 mM of salts at pH 10.0 brings a new perspective on the cultivation of microalgae, and in particular *N. oleoabundans,* and the possible improvement of $CO_2$ transfer in photobioreactors. Its potential as an alternative source for biofuels production is also shown, as lipids also accumulate under those conditions. Lipid accumulation even seemed more pronounced when nitrogen starvation was applied at high pH.

### Example 4: Biomass and fatty acid productivity of *Neochloris oleabundans* under alkaline-saline conditions

#### *Experimental settings*

The methods for producing the microalgal strain and growth medium, as well as the photobioreactor are such as described in Example 1.

By varying the dilution rate between 0.6 and 1.9 d-1, two sets of chemostat experiments were carried out: one at reference pH 8.2 and 3.0% salinity, and another at pH 10.0 and 3.4% salinity. The pH was set by the $CO_2$ supplied via gassing and by the addition of $NaHCO_3$ and $Na_2CO_3$ to the medium. At reference pH, 12 mM $NaHCO_3$ was in equilibrium with 1% v/v $CO_2$. At higher pH, a mixture of 75 mM $NaHCO_3$ and 75 mM $Na_2CO_3$ was applied, while gassing with 0.1% v/v $CO_2$. In this case, the concentration of NaCl in the medium was reduced to 270 mM, as to keep the same osmolarity as in the reference medium.

The cultures were monitored until steady-state was reached. All steady-states were then maintained for 3 to 4 days during which the overflow was collected on ice and harvested every 24 hours for later analysis of biomass dry weight and fatty acids. The microalgae were deprived of nitrogen following some of the nutrient replete steady states, namely after three of the steady-states at reference conditions (dilution rates 0.6 d$^{-1}$, 1.0 d$^{-1}$ and 1.9 d$^{-1}$) and after one steady-state at elevated pH (1.9 d$_{-1}$). Nitrogen depletion was applied by replacing the feed medium by N-free medium, resulting in a gradual but complete depletion of residual nitrogen in the culture broth. Overflow samples were continued to be collected as described above, for 2 days after medium replacement.

#### *Biomass productivity and observed yield on light energy*

The growth medium contained sufficient nutrients to sustain the biomass concentrations obtained in the chemostats. Hence, light availability was the limiting factor for growth (at given temperature, salinity and pH), determining the biomass concentration at the different dilution rates in steady-state. The volumetric biomass productivity ($P_{x,vol}$, $g_{DW}$ L$^{-1}$ d$^{-1}$) was determined daily for each of the steady-states by multiplying the set dilution rate and the measured dry weight biomass concentration in the overflow ($C_x$, $g_{DW}$ L$^{-1}$). Under nitrogen depletion, since there was equilibrium with 1% v/v $CO_2$. At higher pH, a mixture of 75 mM 166 $NaHCO_3$ and 75 mM $Na_2CO_3$ was applied, while gassing with 0.1% v/v $CO_2$. In this case, the concentration of NaCl in the medium was no longer a steady-state situation, Px,vol and Cx were determined for one 189 time point only, just before harvest. During photoautotrophic growth, the efficiency of light use can be assessed by the observed biomass yield on light energy ($Y_{x/E'obs}$, $g_{DW}$ mol$_{ph-1}$). This yield can be expressed by Equation (1) as the amount of biomass ($g_{DW}$) produced per amount of photons absorbed (mol$_{ph}$):

$$Y_{x/E,obs} = \frac{P_{x,vol}}{PF_{abs,vol}} = \frac{C_x \times \mu}{PFD_{abs} \times \left(\frac{A_R}{V_R}\right) \times 0.001 \times 24 \times 3600 \times 10^{-6}} \quad (1)$$

$PF_{abs,vol}$ ($mol_{ph}$ $L_{-1}$ $d_{-1}$) is the photon flux absorbed in a unit volume of the system, i.e. the volumetric photon absorption rate. $PF_{abs,vol}$ corresponds to the product between the photon flux density absorbed by the biomass ($PFD_{abs}$, according to Equation (2)) and the illuminated surface area ($A_R$, $m_2$) to volume ($V_R$, $m_3$) ratio. In Equation (2), $PFD_{out,biomass}$ corresponds to the light passing through the PBR when biomass is present, measured at the PBR's backside at a fixed reference position. Accordingly, $PFD_{out,medium}$ is the light measured at the same reference position with solely medium present.

$$PFD_{abs} = PFD_{in} \times \left(1 - \frac{PFD_{out,biomass}}{PFD_{out,medium}}\right) \quad (2)$$

### Specific light absorption rate

The specific light absorption rate ($r_{E/x}$, $mol_{ph}$ $g_{DW-1}$ $d_{-1}$) was also determined for each dilution rate in steady-state, as the quotient between the volumetric photon absorption rate in the photobioreactor and the biomass concentration, as described by Equation (3):

$$r_{E/x} = \frac{PF_{abs,vol}}{C_x} \quad (3)$$

### Fatty acids quantification

Fatty acid methyl esters (FAMEs) were identified and quantified.
Fatty acid productivity ($P_{FA}$, $g_{FA}$ $L_{-1}$ $d_{-1}$) was determined at each dilution rate in steady state, according to Equation 4:

$$P_{FA} = P_x \times f_{FA/x} \quad (4)$$

$f_{FA/x}$ corresponds to the weight fraction of fatty acids in the biomass.
Under nitrogen depletion, since there was no longer a steady-state situation, $P_{FA}$ was determined for one time point only, just before harvest.

### Visualization of intracellular neutral lipid bodies

The accumulation of intracellular lipid bodies by the microalgae was checked using Confocal Laser Scanning Microscopy (CLSM). The lipophilic fluorescent dye BODIPY 505/515 (4,4-difluoro-1,3,5,7-tetramethyl-4-bora-3a,4a-diaza-sindacene; Invitrogen Molecular Probes, Carlsbad, CA) was used for staining the cells.
Slides were prepared for observation under a confocal microscope (Carl Zeiss B.V., Sliedrecht, The Netherlands).

### Effect of the cultivation conditions on biomass concentration and productivity

The optimum conditions for the production of *N. oleoabundans* were evaluated in a photobioreactor operated as a chemostat. We performed two sets of experiments (one at pH 8.2 and another at pH 10.0) in which different dilution rates were applied to assess the maximal productivity at a given incident light intensity. When operating a chemostat under light limiting conditions, the biomass concentration obtained in steady-state is set by the dilution rate applied. This is because the dilution rate equals the specific growth rate of the cells in steady-state. The latter requires a certain amount of light to be available per cell (higher growth rates require more light energy) and thus dictates the attainable biomass concentration at a given incident light intensity.

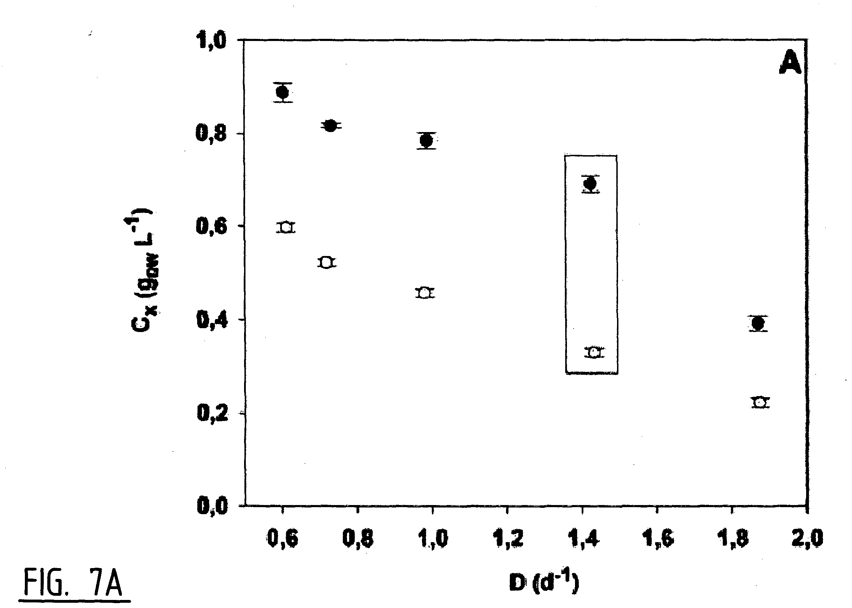

A decrease of the steady-state biomass concentration ($C_x$) with increasing dilution rates was observed, varying from 0.89 to 0.39 $g_{DW}$ $L_{-1}$ at pH 8.2 and from 0.60 to 0.22 $g_{DW}$ $L_{-1}$ at pH 10.0 (Figure 7A). In both cases, the highest $C_x$ was obtained at the lowest dilution rate applied (0.6 $d_{-1}$). The light passing through the system ($PFD_{out,biomass}$) and the average light intensity in the photobioreactor ($PFD_{avg}$) in general increased with increasing D (Table 7), as expected due to the decrease in biomass concentration.

*Table 7: Influence of dilution rate (D, $d_{-1}$) on light parameters and $OD_{680}$ to $OD_{735}$ ratio. At a given incident photon flux density ($PFD_{in}$), PFDout,biomass is the light passing through the photobioreactor, PFDavg is the average light intensity available per cell and $PF_{abs}$ is the light absorbed in the system.*

| # | D ($d^{-1}$) | $PFD_{in}$ $\mu mol_{ph} m^{-2} s^{-1}$ | $PFD_{out, biomass}$ $\mu mol_{ph} m^{-2} s^{-1}$ | $PFD_{avg}$ $\mu mol_{ph} m^{-2} s^{-1}$ | $PFD_{abs}$ $\mu mol_{ph} m^{-2} s^{-1}$ | $OD_{680}/OD_{735}$ |
|---|---|---|---|---|---|---|
| pH 8.2 | 0.6±0.02 | 427 | 48±0.88 | 173±0.88 | 55 | 2.01±0.01 |
| | 0.7±0.01 | 435 | 70±2.10 | 199±3.87 | 52 | 1.68±0.03 |
| | 1.0±0.01 | 435 | 78±0.19 | 208±0.19 | 51 | 1.92±0.02 |
| | 1.4±0.03 | 433 | 33±1.91 | 153±2.72 | 58 | 2.46±0.03 |
| | 1.4±0.01 | 425 | 38±2.74 | 160±3.64 | 56 | 2.52±0.07 |
| | 1.9±0.004 | 425 | 120±1.79 | 241±1.43 | 44 | 2.08±0.03 |
| pH 10.0 | 0.6±0.01 | 442 | 114+6.51 | 242±5.43 | 46 | 1.85±0.05 |
| | 0.7+0.004 | 442 | 132±5.10 | 257±3.98 | 43 | 1.8±0.01 |
| | 1.0±0.01 | 442 | 140±6.52 | 263±4.96 | 42 | 1.91±0.06 |
| | 1.4±0.01 | 442 | 166±8.04 | 282±5.72 | 38 | 1.98±0.07 |
| | 1.9±0.03 | 442 | 231±2.32 | 325±1.46 | 27 | 1.90±0.03 |

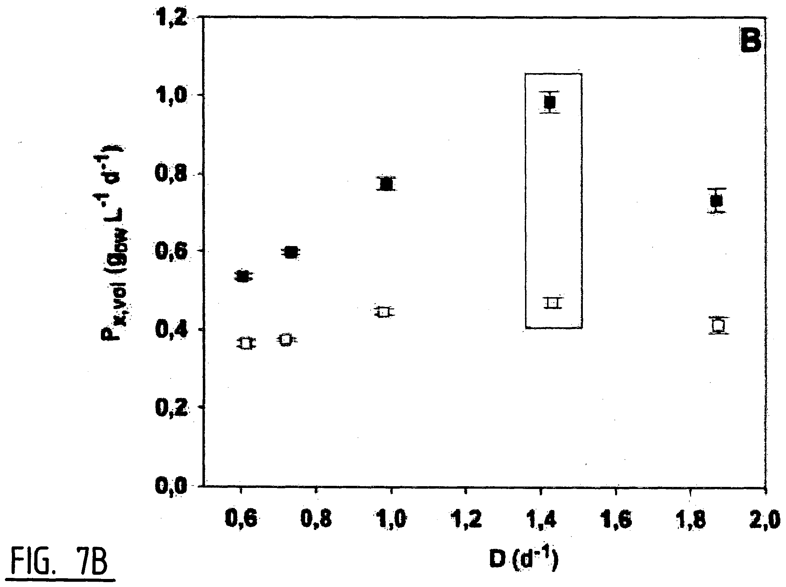

Interestingly, an exception to this trend was observed for the reference pH at D=1.4 $d_{-1}$. Under these conditions, light absorption was the highest of all experiments (experiment performed in separate duplicate runs with similar results, see Table 1), even though the steady-state biomass concentration was markedly lower than those observed for lower dilution rates (Figure 7A, within figure 7 steady state biomass concentration Cx as function of different diluation rates). This higher light absorption was the result of a higher pigmentation of the cells. This is reflected in the significantly higher ratio between $OD_{680}$ and $OD_{735}$ (Table 7), which is an indicator of the chlorophyll content of the biomass. It is not clear though what might be the underlying mechanism governing this trend-breaking adaptation state. We did not observe the same phenomenon at pH 10.0 (Figure 7A and Table 7), and light absorption data of similar experiments in literature do not show such a phenomenon.

At all dilution rates, the biomass concentration at pH 10.0 was lower than at pH 8.2 (Figure 7A). This suggests that at pH 10.0 the microalgae need to be exposed to a higher light fraction to realise the same specific growth rate as the cells growing at pH 8.2. The lower biomass concentrations obtained at pH 10.0 thus resulted in lower biomass productivities for each dilution rate (Figure 7B). For both sets of conditions, an optimal combination of specific growth rate and biomass concentration was observed, resulting in maximal volumetric biomass productivity (Figure 7B) .

The fraction of energy lost to maintenance is lowered when the biomass concentration is reduced, but this increases the culture fraction in which the light intensity is oversaturating for photosynthesis (on the condition that the incident light intensity is oversaturating). Under oversaturating conditions, photosynthesis occurs at maximum rate and the microalgae dissipate the excess of absorbed light energy as heat. At very low biomass concentrations, a large share of the supplied light energy is therefore lost to heat production. In this case, also only a small share is left for biomass formation (sub-optimal productivities at high dilution rates in Figure 7B). Hence, there is an optimum biomass concentration for which biomass is produced most efficiently with regard to the supplied light energy. Maximum $P_{x,vol}$ values of 0.98 $g_{DW}$ $L_{-1}$ $d_{-1}$ at reference conditions and 0.47 $g_{DW}$ $L_{-1}$ $d_{-1}$ at higher pH were obtained, both at a dilution rate of 1.4 $d_{-1}$ (Figure 7B). For the present experimental set-up, this corresponds to optimal biomass concentrations of 0.69 $g_{DW}$ $L_{-1}$ and 0.33 $g_{DW}$ $L_{-1}$, respectively (Figure 7A).

### *Biomass yield on light energy*

While the biomass yield on incident light is a very useful parameter for comparison of photobioreactors performance (e.g. different designs, orientation and location), a detailed understanding of the biological conversion of light energy into biomass requires the yield to be expressed per absorbed light energy ($Y_{x/E,obs}$, Equation 1).

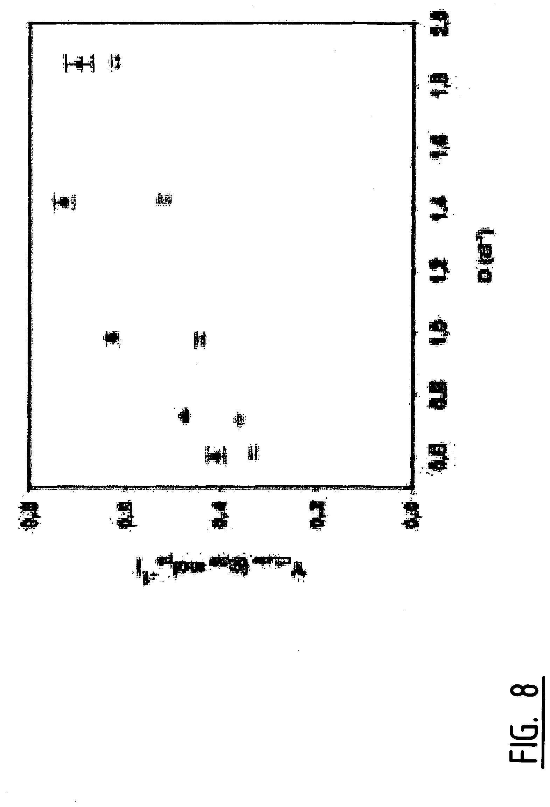

For the reference experiments, $Y_{x/E,obs}$ increased with the dilution rate until 1.4 $d_{-1}$, reaching a value of 0.73 $g_{DW}$ $mol_{ph-1}$, and subsequently decreased at the highest dilution rate applied to 0.70 $g_{DW}$ $mol_{ph-1}$ (Figure 8).

At dilution rates below the optimum an increasing portion of the supplied 308 light energy must be used to provide additional ATP for maintenance purposes, either by respiration or possibly by cyclic photophosphorylation. As a consequence, less light energy is available for growth and the biomass yield on light energy drops. The dilution rate at which the volumetric productivity was maximal at pH 8.2 exactly corresponds to the dilution rate where the biomass yield on light energy was the highest. This is as expected since the volumetric light absorption is roughly constant and only the productivity is changing. In contrast, $Y_{x/E,obs}$ did not show a distinct optimum at pH 10.0 and continuously increased with the dilution rate (Figure 8). In this case, the yield on absorbed light reached a value of 0.63 $g_{DW}$ $mol_{ph-1}$ at the highest dilution rate applied (1.9 $d_{-1}$), very close to the one obtained at pH 8.2 at that same dilution rate. All biomass yields at higher pH were lower than those obtained for the same dilution rates at reference pH.

In theory, the lower yields at pH 10.0 could be explained by elevated heat dissipation due to a higher level of oversaturation, by higher energy requirements for biomass formation or for cell maintenance, or a combination of the three. We suggest a higher energy requirement either for biomass formation or for cell maintenance to be the most probable high pH related effects.

The basis for our hypothesis is the relation between the specific light absorption rate ($r_{E/x}$, $mol_{ph}$ $g_{DW-1}$ $d_{-1}$, Equation 3) and the specific growth rate ($\mu$, $d_{-1}$).

This relation can be described by the model of Pirt (1995) and translated into Equation (5).

$$r_{E/x} = \frac{\mu}{Y_{x/E,true}} + m_{E/x} \ (5)$$

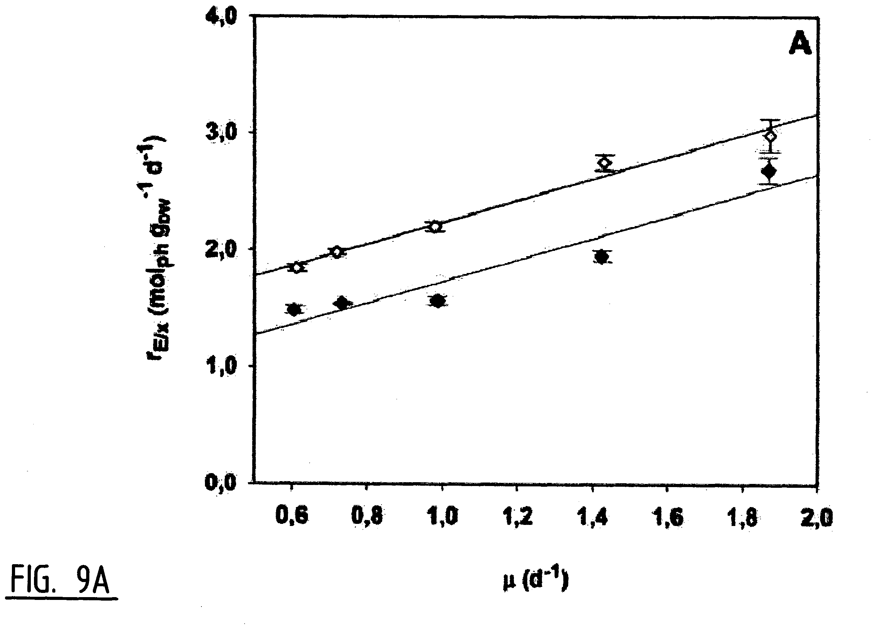

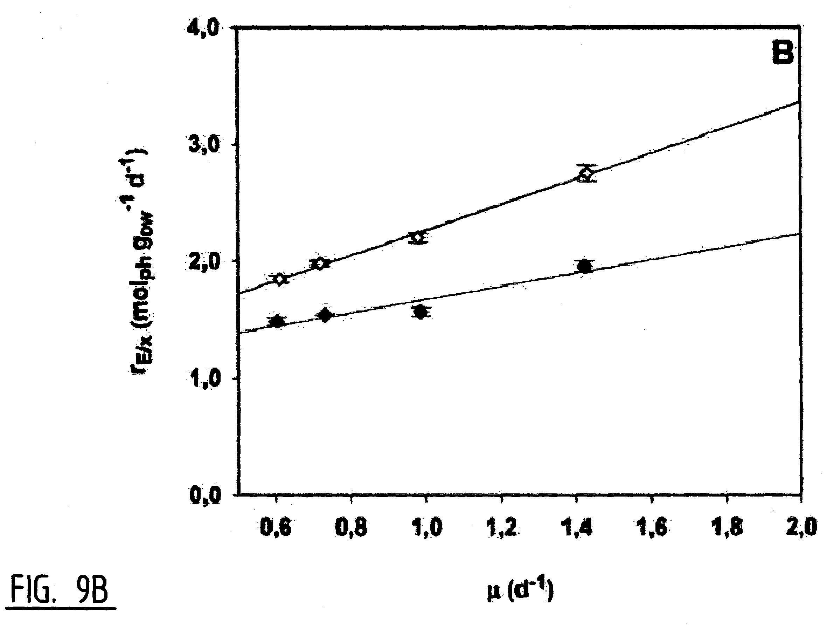

In Equation (5), $Y_{x/E,true}$ is the biomass yield on light energy (i.e. energy required for formation of new biomass, $g_{DW}$ $mol_{ph-1}$) and $m_{E/x}$ is the maintenance requirement ($mol_{ph}$ $g_{DW-1}$ $d_{-1}$). The model assumes that the fraction of light used by the microalgae for biomass formation increases with $\mu$ at a constant yield, whilst a fixed amount of light energy per cell is required for cellular maintenance. Plotting the linear regression of $\mu$ against $r_{E/x}$ for all steady-states therefore should result in a straight line of which the slope corresponds to the inverse of $Y_{x/E,true}$ and the offset to $m_{E/x}$. A non-linear curve at pH 8.2 (Figure 9A). The lines obtained for both experiments were in this case quite similar, both resulting in a $Y_{x/E,true}$ of 1.09 $g_{DW}mol_{ph-1}$. The maintenance requirement was however higher at pH 10.0 (1.3 molph $g_{DW-1}d_{-1}$ in comparison with 0.8 molph $g_{bio-1}$ $d_{-1}$ at pH 8.2), which could explain the lower values of $Y_{x/E,obs}$ (Figure 8). But the non-linear behavior at pH 8.2 is very likely caused 345 by a relatively large loss of energy due to non-photochemical quenching (NPQ) at the highest dilution rate applied. In this case, a higher fraction of light is quenched by heat or fluorescence instead of being absorbed. Such an explanation seems reasonable since the overall biomass yield on absorbed light energy decreased substantially at this dilution rate, while the average light intensity inside the photobioreactor was highest of all dilution rates applied at pH 8.2 ($PFD_{avg}$, Table 7). This average light intensity was exceeded at all experiments at pH 10.0 (Table 1), especially at the end (D=1.9 $d_{-1}$), so the higher $r_{E/x}$ values could also be due to an increase in NPQ. Moreover, higher $r_{E/x}$ reinforces the idea that individual cells require a higher light availability to grow at the same rates as at pH 8.2. But paradoxically, a non-linear relation between $r_{E/x}$ and D was not observed. In any case, if we assume that in both experimental sets substantial energy losses occurred at D=1.9 $d_{-1}$ due to oversaturation and eliminate the correspondent data points, both regressions will then be linear (Figure 9B). This alternative scenario gives a $m_{E/x}$ of 1.1 $mol_{ph}$ $g_{DW-1}$ $d_{-1}$ at pH 8.2 and of 1.2 $mol_{ph}$ $g_{DW-1}$ $d_{-1}$ at pH 10.0, suggesting that the cultivation pH has hardly an effect on the cellular maintenance requirement. The true biomass yield on absorbed light ($Y_{x/E,true}$) differs substantially though, being then 1.79 $g_{DW}$ $mol_{ph-1}$ at pH 8.2 and 0.92 $g_{DW}$ $mol_{ph-1}$ at pH 10.0. Hence, we give two possible hypotheses for the reduced overall biomass yield at elevated pH (Figure 8): increased energy costs for maintenance (Figure 9A) or for biomass formation (Figure 9B) .

| Organism | $Y_{x,E,ph}$ $(g_{DW}\ mol_{ph}^{-1})$ | $PFD_{in}$ $(\mu mol_{ph}\ m^{-2}\ s^{-1})$ | $C_x$ $(g_{DW}\ L^{-1})$ | pH | Reference |
|---|---|---|---|---|---|
| *Neochloris oleoabundans* | 0.73[a]/0.66[b] | 425 | 0.69 | 8.2 | This study |
| *Neochloris oleoabundans* | 0.63[a]/0.31[b] | 442 | 0.33 | 10.0 | This study |
| *Chlorella sorokiniana* | 1.00[b] | 2100 | 2.10 | 6.7 | Cuaresma et al. (2009) |
| *Dunaliella tertiolecta* | 0.65[b] | 990 | 1.40-3.80 | 7.8 | Züffers et al. (2010) |
| *Chlamydomonas reinhardtii* | 0.80[b] | 1500 | 1.20[d] | 7.0 | Kliphuis et al. (2010a) |
| *Neochloris oleoabundans* | 0.71[b] | 270 | 0.90 | 7.5 | Pruvost et al. (2009) |
| *Neochloris oleoabundans* | 1.04[b] | 200 | 0.40 | 7.8 | Sousa et al. (2012) |
| *Chlamydomonas reinhardtii* | 0.51[b,c] | 1000 | 1.10 | 7.5 | Takache et al. (2010) |
| *Chlamydomonas reinhardtii* | 0.72[b,c] | 500 | 0.60 | 7.5 | Takache et al. (2010) |
| *Chlamydomonas reinhardtii* | 1.11[b,c] | 110 | 0.35 | 7.5 | Takache et al. (2010) |

[a] $Y_{x,E,ph}$ based on $PFD_{abs}$.

[b] $Y_{x,E,ph}$ based on $PFD_{in}$.

[c] Calculated based on Flat-torus PBR experimental data.

[d] $C_x$ obtained during batch cultivation at the maximum $PFD_{in}$ applied.

*Table 8: Comparison of experimental biomass yields on light energy found in this study with several from other microalgae, at different light intensities, optimal biomass densities and cultivation pH.*

### Effect of nitrogen supply on biomass production and fatty acid accumulation

*N. oleoabundans* was analyzed for its total fatty acid content and composition during all steady-states in nitrogen-replete medium and two days after the medium was replaced with nitrogen-free medium. The biomass productivities, fatty acid content and productivities under nitrogen-replete and depletion conditions are summarized in Table 8.

Despite the pH applied, the total fatty acid content didn't vary substantially within the different dilution rates under nitrogen-replete conditions, being around 8% at pH 8.2 and increasing to 15% at pH 10.0. This higher fatty acid accumulation during growth at higher pH may also explain the lower biomass yields under that condition (sub-section 3.2). It suggests that besides the need for extra light energy for biomass formation or for cell maintenance under less optimal conditions, the cells spend part of that energy for storing more fatty acids.

The constant fatty acid content observed under nitrogen-replete conditions suggests a constant biomass composition. Therefore, the fatty acid productivity followed the same trend as the biomass productivity, by increasing with the dilution rate until reaching a maximum. A maximal volumetric fatty acid productivity of 75.20 $mg_{FA}$ $g_{-1}$ $L_{-1}$ at D=1.4 $d_{-1}$ was found at pH 8.2 and of 66.55 $mg_{FA}$ $g_{-1}$ $L_{-1}$ at pH 10.0 at the same dilution rate.

The higher fatty acid contents obtained at pH 10.0 in general corresponded to higher productivities. Even so, the highest fatty acid productivity was found at pH 8.2, due to the much higher biomass productivity at the corresponding optimal dilution rate.

It has been shown before that nitrogen limitation causes *N. oleoabundans* cells to stop dividing but, simultaneously, triggers lipid/fatty acid accumulation.

In this case, applying nitrogen depletion therefore caused a decrease of the biomass productivity and a consequent increase of the fatty acid content and productivity. Fatty acids accumulated up to 18% (w/w) when nitrogen depletion was applied at pH 8.2 and their productivity went up to 67 $mg_{FA}$ $L_{-1}$ $d_{-1}$. On the other hand, the total fatty acid content of *N. oleoabundans* was 29% (w/w) at pH 10.0, corresponding to a productivity of 112.41 $mg_{FA}$ $L_{-1}$ $d_{-1}$, the highest found.

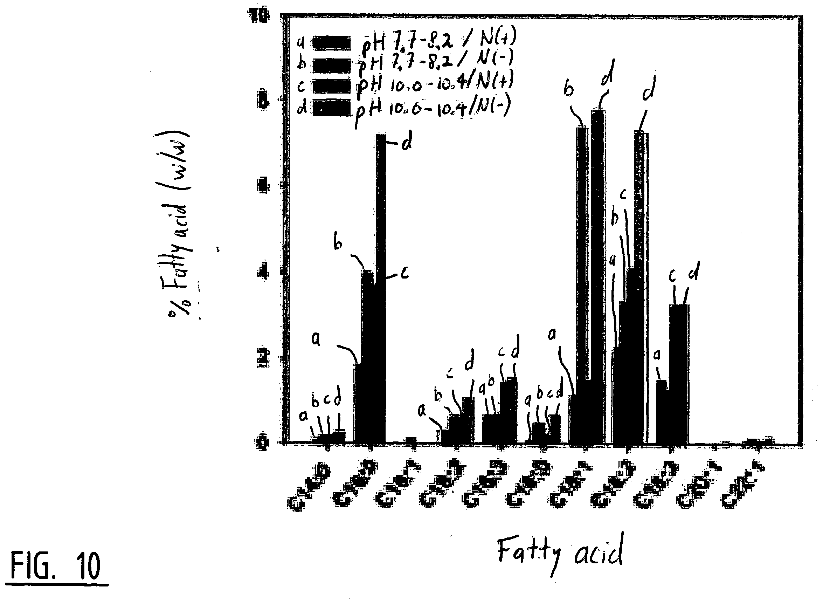

An elevated pH enhances N-starvation-induced fatty acid accumulation. Additionally, pH-related stress by itself can cause an increase on the fatty acid accumulation, being independent from nitrogen-stress. In terms of Neochloris fatty acid composition no variation between the dilution rates applied and pH treatments was observed. An increase of the C16:0 and C18:1 fatty acids upon nitrogen depletion was was observed, especially when associated with elevated pH. In comparison to that, the content of more unsaturated fatty acids was less substantial (Figure 10), which is more favorable for the preparation of biofuels. The more saturated character of the fatty acids involved requires less hydrogenation during the process. Especially the neutral lipids are of interest, since they are easier to extract and separate.

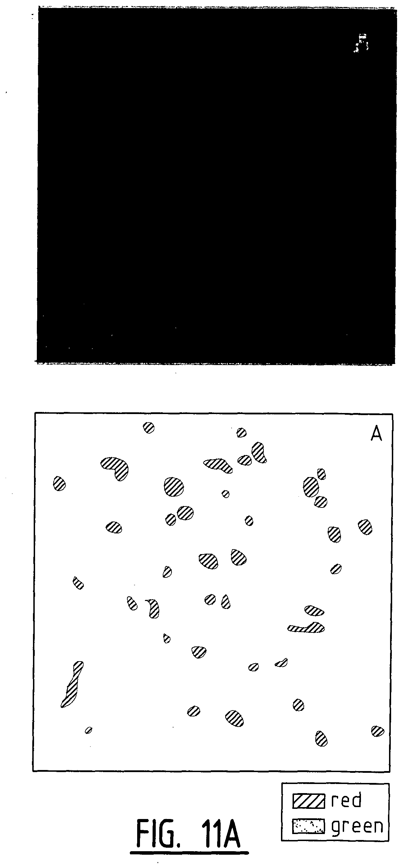

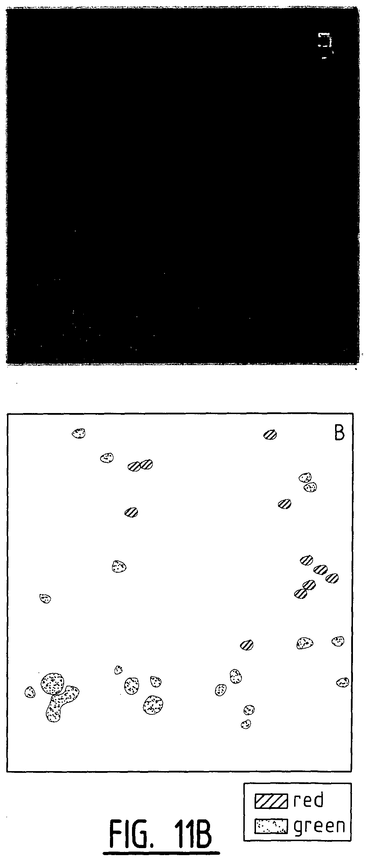

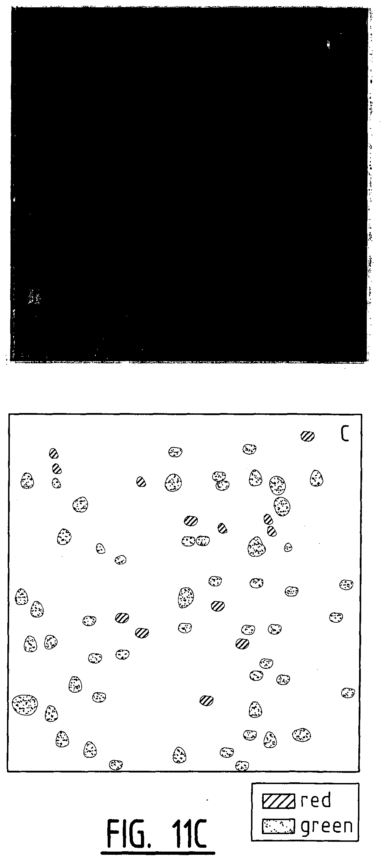

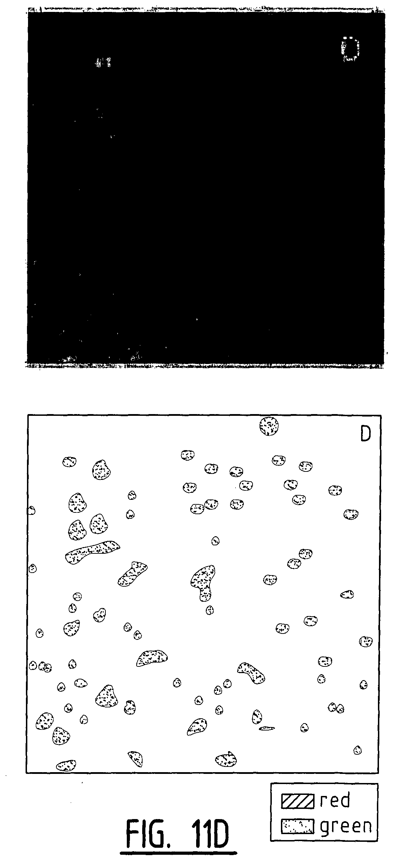

During the course of both sets of experiments the cells were stained with BODIPY 505/515 and the visualization of neutral lipid bodies was possible, supporting our results. The cells showed green fluorescent spherical bodies, either with or without nitrogen in the medium. The accumulation was more evident after the switch to medium without nitrogen (Figure 11) .

*Table 9: Total fatty acid content (% FA w/w) and productivity ($P_{FA}$, $g_{FA}$ $L_{-1}$ $d_{-1}$) of N. oleoabundans at different dilution rates (D, $d_{-1}$) at reference conditions (pH 8.2) and higher pH (pH 10.0). Results without nitrogen limitation are represented by N(+) and with nitrogen depletion by N(-). Values of biomass concentration (Cx, $g_{DW}$ $L_{-1}$) and productivity ($P_{x,vol}$, $g_{DW}$ $L_{-1}$ $d_{-1}$) under the different conditions are also given.*

| | D ($d^{-1}$) | $C_x$ ($G_{DW}$ $L^{-1}$) | $P_{x,vol}$ ($G_{DW}$ $L^{-1}$ $d^{-1}$) | | Total FA (%w/w) | | PFA, ($mg_{FA}$ $L^{-1}$ $d^{-1}$) | |
|---|---|---|---|---|---|---|---|---|
| | | | N(+) | N(-) | N (+) | N(-) | N(+) | N(-) |
| **pH 8.2** | 0.6±0.02 | 0.89 | 0.54±0.02 | 0.33±0.01 | 8.74±0.17 | 18.18±0.01 | 46.85±0.93 | 60.52±0.05 |
| | 0.7±0.01 | 0.82 | 0.60±0.01 | - | 8.33±0.002 | - | 49.74±0.01 | - |
| | 1.0±0.01 | 0.78 | 0.77±0.02 | 0.32±0.01 | 7.53±0.01 | 18.52±0.01 | 58.30±0.11 | 64.56±0.03 |
| | 1.4±0.03 | 0.69 | 0.98±0.03 | - | 7.65 | - | 75.20 | - |
| | 1.9±0.004 | 0.39 | 0.73±0.03 | 0.37±0.02 | 7.13±0.01 | 18.11±0.01 | 52.07±0.09 | 67.54±0.02 |
| **pH 10.0** | 0.6±0.01 | 0.60 | 0.37±0.01 | - | 14.98±0.01 | - | 54.85±0.01 | - |
| | 0.7±0.004 | 0.52 | 0.38±0.00 4 | - | 14.89±0.18 | - | 55.84±0.67 | - |
| | 1.0±0.01 | 0.46 | 0.45±0.01 | - | 13.92±0.40 | - | 62.07±1.78 | - |
| | 1.4±0.01 | 0.33 | 0.47±0.01 | - | 14.19±0.03 | - | 66.55±0.12 | - |
| | 1.9±0.03 | 0.22 | 0.41±0.02 | 0.33±0.07 | 14.94±0.01 | 29.20±0.04 | 61.42±0.02 | 112.41±0.17 |

Considering the above, growing *N. oleoabundans* in alkaline-saline conditions such as in the method according to the present invention, plus nitrogen depletion, brings an advantage in boosting lipid production for microalgal biofuels. Such process can also improve $CO_2$ transfer in photobioreactors, as less energy will be needed. Further, growing microalgae under alkaline-saline for sustainable production of lipids, carbohydrates and other compounds according to the method of the present invention therefore contributes to cost effective use of microalgae as feedstock for food, fuel, chemicals, and feed at industrial scale. An additional advantage of microalgal growth and lipid production under alkaline-saline conditions such as recited in the present invention is that in these conditions act as an additional hurdle for microbial contamination during the process.

The present invention is by no means limited to the above described preferred embodiments thereof. The rights sought are defined by the following claims within the scope of which many modifications can be envisaged.

**Claims**

1. Method for obtaining algal biomass with increased lipid accumulation, comprising the step of culturing photoautotrophic freshwater micro-algae in a culturing medium having a pH above 9, wherein lipid accumulation is increased compared to cultivation of such algal biomass at a pH below 8, wherein the medium is a saline medium, wherein the cultivating medium comprising NaCl in a concentration of at least 200 mM, wherein the algae are cultivated under conditions of nutrient limitation, wherein the nutrient limitation conditions comprise nitrogen limitation.

2. Method according to claim 1, wherein the pH of the medium is in the range of 9-11 and most preferably 10-11.

3. Method according claim 1 or 2, wherein the algal biomass comprises algal cells, preferably lipids comprised by the algal cells.

4. Method according to claim 3, wherein the algae are lipid-rich algae.

5. Method according to any of claims 1 to 4, wherein the algae are of the species *Neochloris oleoabundans.*

6. Method according to claim 1, wherein the medium comprises seawater and/or artificial seawater.

7. Method according to any one of claims 1 to 6, the cultivating medium comprising alkali metal chlorine salts, being NaCl, in a concentration providing the medium with a higher salinity than freshwater, preferably in a concentration of at least 200, 270 or 400 mM.

8. Method according to any one of claims 1 to 7, wherein the pH of the culturing medium is regulated by an equilibrium of carbon dioxide, bicarbonate and carbonate.

9. Method according to claim 8, wherein bicarbonate and carbonate ions are present in an amount sufficient to buffer the medium at a pH of at least 9, 9.5, 10, 10.5 or 11.

10. Method according to any one of claims 1 to 9, wherein carbon dioxide is supplied for carbon dioxide transfer to the medium in a gas which comprises 0.01%-20% (v/v) carbon dioxide.

11. Method according to any one of claims 1 to 10, wherein the cultivating medium comprises ions of a soluble salt, the concentration being such that no significant precipitation of the ions at the given pH level occurs.

12. Method according to claim 11, wherein the cultivating medium comprises Ca and Mg ions, wherein the concentration of Ca ions is between 0.001 and 0.15 mM and the ratio Ca:Mg is between 1:5 and 1:30, preferably 1:10 to 1:20, more preferably 1:15.

13. Method according to any one of claims 1 to 12, further comprising measuring the pH and/or the ion concentration.

**Patentansprüche**

1. Verfahren zur Gewinnung von Algenbiomasse mit erhöhter Lipidakkumulation, umfassend den Schritt des Kultivierens photoautotropher Süßwasser-Mikroalgen in einem Kulturmedium mit einem pH-Wert über 9, wobei die Lipidakkumulation erhöht ist im Vergleich zur Kultivierung solcher Algenbiomasse bei einem pH-Wert unter 8, wobei das Medium ein Salzmedium ist, wobei das Kulturmedium NaCl in einer Konzentration von mindestens 200 mM enthält, wobei die Algen unter Bedingungen der Nährstoffbegrenzung kultiviert werden, wobei die Nährstoffbegrenzungsbedingungen eine Stickstoffbegrenzung umfassen.

2. Verfahren nach Anspruch 1, wobei der pH-Wert des Mediums im Bereich von 9 - 11 und am meisten bevorzugt 10 - 11 liegt.

3. Verfahren nach Anspruch 1 oder 2, wobei die Algenbiomasse Algenzellen enthält, vorzugsweise von den Algenzellen umfasste Lipide.

4. Verfahren nach Anspruch 3, wobei die Algen lipidreiche Algen sind.

5. Verfahren nach einem der Ansprüche 1 bis 4, wobei die Algen von der Spezies *Neochloris oleoabundans* sind.

6. Verfahren nach Anspruch 1, wobei das Medium Meerwasser und / oder künstliches Meerwasser umfasst.

7. Verfahren nach einem der Ansprüche 1 bis 6, wobei das Kulturmedium Chloralkalimetallsalze, nämlich NaCl, in einer Konzentration enthält, die dem Medium einen höheren Salzgehalt als Süßwasser verleihen, vorzugsweise in einer Konzentration von mindestens 200, 270 oder 400 mM.

8. Verfahren nach einem der Ansprüche 1 bis 7, wobei der pH-Wert des Kulturmediums durch ein Gleichgewicht von Kohlendioxid, Bicarbonat und Carbonat reguliert wird.

9. Verfahren nach Anspruch 8, wobei Bicarbonat- und Carbonationen in einer Menge vorhanden sind, die ausreicht, um das Medium bei einem pH-Wert von mindestens 9, 9,5, 10, 10,5 oder 11 zu puffern.

10. Verfahren nach einem der Ansprüche 1 bis 9, wobei Kohlendioxid zur Kohlendioxidübertragung an das Medium in einem Gas zugeführt wird, das 0,01% - 20% (v / v) Kohlendioxid umfasst.

11. Verfahren nach einem der Ansprüche 1 bis 10, wobei das Kulturmedium Ionen eines löslichen Salzes enthält, wobei die Konzentration derart ist, dass keine signifikante Ausfällung der Ionen bei dem gegebenen pH-Wert stattfindet.

12. Verfahren nach Anspruch 11, wobei das Kulturmedium Ca- und Mg-Ionen umfasst, wobei die Konzentration der Ca-Ionen zwischen 0,001 und 0,15 mM liegt und das Verhältnis Ca:Mg zwischen 1:5 und 1:30, vorzugsweise 1:10 bis 1:20, besonders bevorzugt 1:15 liegt.

13. Verfahren nach einem der Ansprüche 1 bis 12, ferner umfassend das Messen des pH-Wertes und / oder der Ionenkonzentration.

**Revendications**

1. Procédé d'obtention de biomasse algale avec une accumulation de lipides augmentée, comprenant l'étape de cultiver des algues photoautotrophes d'eau douce dans un milieu de culture ayant un pH supérieur à 9, dans lequel l'accumulation de lipides est augmentée par rapport à la cultures d'une telle biomasse d'algues à un pH inférieur à 8, dans lequel le milieu est un milieu salin, dans lequel le milieu de culture comprend du NaCl à une concentration d'au moins 200 mM, dans lequel les algues sont cultivées dans des conditions de limitation en éléments nutritifs, dans lequel les conditions de limitation en éléments nutritifs comprennent la limitation en azote.

2. Procédé selon la revendication 1, dans lequel le pH du milieu est compris dans la plage allant de 9 à 11 et le plus préférablement de 10 à 11.

3. Procédé selon la revendication 1 ou 2, dans lequel la biomasse algale comprend des cellules algales, de préférence des lipides compris dans les cellules algales.

4. Procédé selon la revendication 3, dans lequel les algues sont des algues riches en lipides.

**5.** Procédé selon l'une quelconque des revendications 1 à 4, dans lequel les algues sont de l'espèce Neochloris oleoabundans.

**6.** Procédé selon la revendication 1, dans lequel le milieu comprend de l'eau de mer et/ou de l'eau de mer artificielle.

**7.** Procédé selon l'une quelconque des revendications 1 à 6, le milieu de culture comprenant des sels de chlore et de métal alcalin, étant du NaCl, en une concentration donnant au milieu une salinité supérieure à celle de l'eau douce, de préférence à une concentration d'au moins 200 mM, 270 mM ou 400 mM.

**8.** Procédé selon l'une quelconque des revendications 1 à 7, dans lequel le pH du milieu de culture est régulé par un équilibre de dioxyde de carbone, de bicarbonate et de carbonate.

**9.** Procédé selon la revendication 8, dans lequel les ions bicarbonate et carbonate sont présents en une quantité suffisante pour tamponner le milieu à un pH d'au moins 9, 9,5, 10, 10,5 ou 11.

**10.** Procédé selon l'une quelconque des revendications 1 à 9, dans lequel du dioxyde de carbone est fourni pour le transfert de dioxyde de carbone dans le milieu dans un gaz qui comprend de 0,01% à 20% (v/v) de dioxyde de carbone.

**11.** Procédé selon l'une quelconque des revendications 1 à 10, dans lequel le milieu de culture comprend des ions d'un sel soluble, la concentration étant telle qu'il ne se produit aucune précipitation significative des ions au pH donné.

**12.** Procédé selon la revendication 11, dans lequel le milieu de culture comprend des ions Ca et Mg, dans lequel la concentration en ions Ca est comprise entre 0,001 mM et 0,15 mM et le rapport Ca:Mg est compris entre 1:5 et 1:30, de préférence entre 1:10 et 1:20, et vaut plus préférablement 1:15.

**13.** Procédé selon l'une quelconque des revendications 1 à 12, comprenant en outre la mesure du pH et/ou de la concentration en ions.

FIG. 1

FIG. 2

FIG. 3A

EP 2 737 049 B1

FIG. 3B

FIG. 4A

FIG. 4B

FIG. 5A

FIG. 5B

EP 2 737 049 B1

FIG. 6A

FIG. 6B

FIG. 6C

FIG. 6D

32

FIG. 7A

FIG. 7B

EP 2 737 049 B1

FIG. 8

FIG. 9A

EP 2 737 049 B1

FIG. 9B

FIG. 10

FIG. 11A

red
green

FIG. 11B

| | |
|---|---|
| ▨ red | |
| ▧ green | |

FIG. 11C

| | red |
| | green |

FIG. 11D

| | |
|---|---|
| ▨ red | |
| ▨ green | |

**REFERENCES CITED IN THE DESCRIPTION**

**Non-patent literature cited in the description**

- **MAZZUCA SOBCZUK et al.** *Journal of Chemical Technology and Biotechnology,* October 2009, vol. 85 (1), 100-108

- **VAZQUEZ-DUHALT R. ; G. GRASSI ; G. GOSSE ; G. DOS SANTOS et al.** Oil production from micro-algae under saline stress. *Biomass for Energy and Industry, 5th E.C. Conference, Volume 1, Policy, Environment, Production and Harvesting,* 1990, vol. 1, 1547-1552